# EUROPEAN PATENT APPLICATION

(11) **EP 4 620 936 A1**
(43) Date of publication of application: **24.09.2025**
(21) Application number: 24164604.1
(22) Date of filing: 19.03.2024
(51) Int. Cl.: C07B 59/00, C07D 519/00

(54) **PROCESS FOR THE PREPARATION OF N6-ISOTOPE-LABELED ERGOT ALKALOIDS, IN PARTICULAR LYSERGIC ACID AMIDES AND ERGOPEPTINES**

(71) Applicant: Bundesrepublik Deutschland, vertreten durch den Bundesminister für Wirtschaft und Klimaschutz, dieser vertreten durch den Präsidenten der, 12205 Berlin (DE)
(72) Inventor: HERTER, Sven-Oliver, 12459 Berlin (DE)
(74) Representative: Zimmermann & Partner Patentanwälte mbB

(57) **Abstract**

A process of synthesizing an N⁶-isotope-labeled ergot alkaloid is suggested, comprising: demethylating the ergot alkaloid to obtain a corresponding
N⁶-demethylated norergot alkaloid; remethylating the corresponding N⁶-demethylated norergot alkaloid to obtain the N⁶-isotopically labeled ergot alkaloid; wherein remethylating is carried out with an isotopically labeled methylation reagent.

## Description

### FIELD

The present invention relates to the monitoring of cereals and cereal-based products for residues of mycotoxins by mass spectrometry. In particular, the invention relates to isotope-labeled mycotoxins which can be used as internal standards in a high-performance liquid chromatography mass spectrometric analysis and which can be distinguished from the natural mycotoxins and their metabolites by their mass-to-charge ratio (m/z value).

### BACKGROUND

Providing safe food for an ever-growing world population is one of the great challenges of the 21st century. At 2.8 billion tons per year, cereals are the world's most important staple food. A toxicologically relevant group of mycotoxins are the ergot alkaloids (EAs). These are secondary metabolites produced by several ubiquitous fungi of the genus *Claviceps.* They grow preferentially on rye and wheat, but can also infect other sweet grasses such as triticale, barley, and millet. Toxic ergot alkaloids are introduced into food and feed through the harvest of sclerotia-infested grain and subsequent processing, e.g. in mills.

The chemical structure of the most important ergot alkaloids and their *C⁸* stereoisomers are based on lysergic acid. The most relevant lysergic acid amides can be divided into two distinct groups: simple lysergic acid derivatives, such as ergometrine, and the group of ergopeptines with a tricyclic peptide ring. The corresponding stereoisomers are formed by isomerization at the *C⁸*-carbon atom of the lysergic acid moiety. The substances comprising the *C⁸*-(*R*) configuration are referred to as ergopeptines (e.g., ergotamine) and the corresponding alkaloids comprising the *C⁸*-(*S*) configuration as ergopeptinines (e.g., ergotaminine).

Both the R- and S-forms exhibit different biological activities, with the ergopeptines being more toxic than the ergopeptinines. However, since both epimers are convertible into each other, they both must be quantified in order to determine the degree of contamination and toxicity of a sample. The European Union established maximum levels for certain ergot alkaloids in foodstuffs, as outlined in Commission Regulation (EC) No 2021/1399 amending Regulation No 1881/2006. In 2022, maximum levels were firstly set for 6 priority ergot alkaloids and their epimers: ergocornine, ergometrine, ergocristine, ergotamine, ergosine and ergocryptine and their -inine epimers. Simultaneously, EN 17425 (april 2021) was published by the Technical Committee CEN/TC 275 "Food analysis - Horizontal methods" as the first standardized method for quantifying priority ergot alkaloids using high-performance liquid chromatography with tandem mass spectrometry (HPLC-MS/MS).

In HPLC-MS/MS, the matrix co-eluting with the analyte molecules can directly affect the ionization efficiency, resulting in a reduction of accuracy, precision, and sensitivity, known as matrix effect. To overcome this issue, internal standards (ISTD) can be used. In HPLC-MS/MS, a suitable ISTD is a chemically similar compound to the analyte, with comparable retention time, ionization response, and fragmentation pattern.

Therefore, isotopically (²H, ¹³C, ¹⁵N) labeled ISTD are preferably used for food, environmental and bioanalytical methods to improve quantification. However, in case of the six priority ergot alkaloids and their epimers, isotopically labeled ISTD are not fully commercially available, which limits monitoring of these mycotoxins based on MS. The resulting need is underlined by the reduction of the maximum levels of ergot alkaloids in various cereal products from July in 2024 set by Commission Regulation (EC) No 2023/915.

Various strategies can be employed to address the issue of unavailable isotopically labeled ergot alkaloids. Currently, only the simple lysergic acid derivatives ergometrine and ergometrinine exist as ¹³CD₃-ISTDs, but not the ergopeptines. Synthesizing the cyclic peptide ring and then coupling it with lysergic acid has been undertaken. However, this process of total synthesis is time consuming and complex, resulting in low overall yields and high production costs. Based on the publication by Braun B., Köppen R., Wedler Ch. and Theil F. (2017) "Synthesis of M + 4 Stable Isotopomers of ergometrine and ergometrinine" in Natural Product Communications 12(3): 373-376, a yield of <0.61% was calculated. In view of the lack of a cyclopeptide ring in ergometrine/ergometrinine compared to the other ergot alkaloids, ergometrine/ergometrinine is the most easily accessible for total synthesis. In this publication, however, yields were only given for 5 of the totally required 6 synthesis steps, so that the overall yield of ergometrine/ergometrinine will be even lower than the stated value.

### BRIEF SUMMARY

Against this background, it is the object of the present invention to provide an effective labeling method which is both simple, i.e. comprises only a minimum number of process steps, is thus cheaper to synthesize, and has a significantly increased yield and thus does not suffer from the aforementioned disadvantages.

According to one embodiment, a synthesis is suggested comprising chemically modifying unlabeled native ergot alkaloids with a stable carbon and hydrogen isotope in order to obtain the labeled ergot alkaloids. In particular, the synthesis comprises firstly demethylating the ergot alkaloid and secondly remethylating the ergot alkaloid with an isotopically labeled methyl group. The demethylation, resulting in the corresponding norergot alkaloid, may be followed by purification, e.g. using chromatography. The isotopically labeled methyl group can include any of ¹¹C, ¹²C, ¹³C, ¹⁴C, ¹H, ²H, i.e. D (deuterium), and ³H (tritium). However, tritium, ¹¹C and ¹⁴C are radioactive, with ¹¹C having a half-life of only about 20.3 minutes, their usability as an internal standard is possible, but unlikely due to the necessary precautions.

According to an embodiment, a method for detecting an ergot mycotoxin in a sample is proposed, comprising the following: Mass spectrometric analysis of the sample, wherein the presence and/or amount of a sought-after mycotoxin is quantified using an N⁶ isotope-labeled ergot alkaloid as an internal standard, which is synthesized as described above.

According to an embodiment a N⁶-isotope-labeled ergot alkaloid is suggested for use as internal standard in high-performance liquid chromatography-mass spectrometry (HPLC-MS) analysis of a sample which is potentially contaminated by an ergot alkaloid, wherein the ergot alkaloid is selected from the group consisting of:
a *5R, 8R* configuration of ergocornine, a *5R, 8S* configuration of ergocornine,
a *5R, 8R* configuration of ergometrine, a *5R, 8S* configuration of ergometrine,
a *5R, 8R* configuration of ergocristine , a *5R, 8S* configuration of ergocristine ,
a *5R, 8R* configuration of ergotamine , a *5R, 8S* configuration of ergotamine,
a *5R, 8R* configuration of ergosine, a *5R, 8S* configuration of ergosine,
a *5R, 8R* configuration of α-ergocryptine, a *5R, 8S* configuration of α-ergocryptine,
a *5R, 8R* configuration of β-ergocryptine, and a *5R, 8S* configuration of β-ergocryptine.

According to an embodiment a kit for detection of an ergot mycotoxin by using a mass spectrometry is suggested, the kit comprising at least one N⁶-isotope-labeled ergot alkaloid as synthesized disclosed above. Therein, the N⁶-isotope -labeled ergocornine, ergometrine, ergocristine, ergotamine, ergosine, α-ergocryptine, and/or β-ergocryptine are synthesized as disclosed above by a demethylation of the corresponding native ergot alkaloid and its subsequent remethylation.

According to an embodiment the use of the N⁶-isotope-labeled ergot alkaloids described above as internal standard in a mass spectrometry measurement is suggested. Using said N⁶-isotope-labeled ergot alkaloid comprises the preparation thereof starting from the corresponding alkaloid by the two-step process suggested herein.

As described further below, the proposed synthesis procedure and kit allows the preparation of isotope-labeled standards for all ergot alkaloids and their corresponding epimers, enabling reliable monitoring of all priority ergot alkaloids and their epimers in a standardized procedure based on HPLC-MS or tandem mass spectrometry (MS/MS) analysis.

### BRIEF DESCRIPTION OF THE DRAWINGS

A full and enabling disclosure of the present invention, including the best mode thereof, to one of ordinary skill in the art, is set forth more particularly in the remainder of the description, including reference to the accompanying figures.

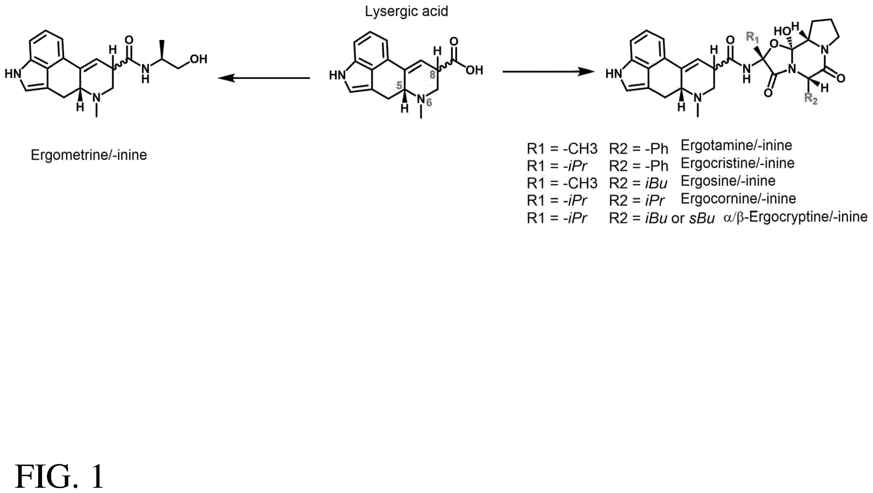

**Fig. 1** shows the structures of the 6 most abundant ergot alkaloids and their *C⁸*-stereoisomers which are based on the structure of lysergic acid.

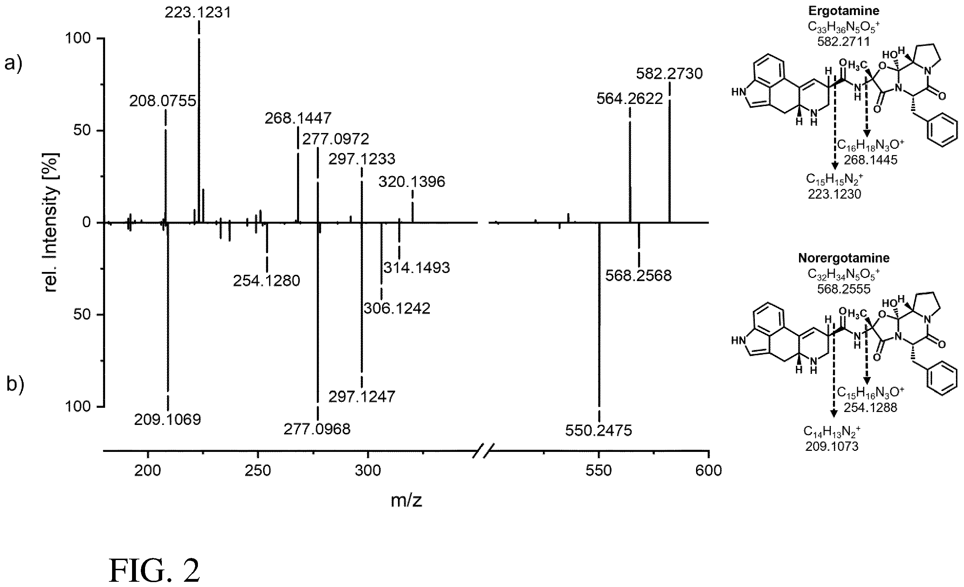

**Fig. 2** Positive electrospray ionization high-resolution tandem mass spectra [M+H]⁺ of **a)** ergotamine and **b)** norergotamine. The structures are displayed on the right-hand side along with the theoretical exact masses for the molecular ion [M+H]⁺ and major fragment ions containing the *N⁶*-atom.

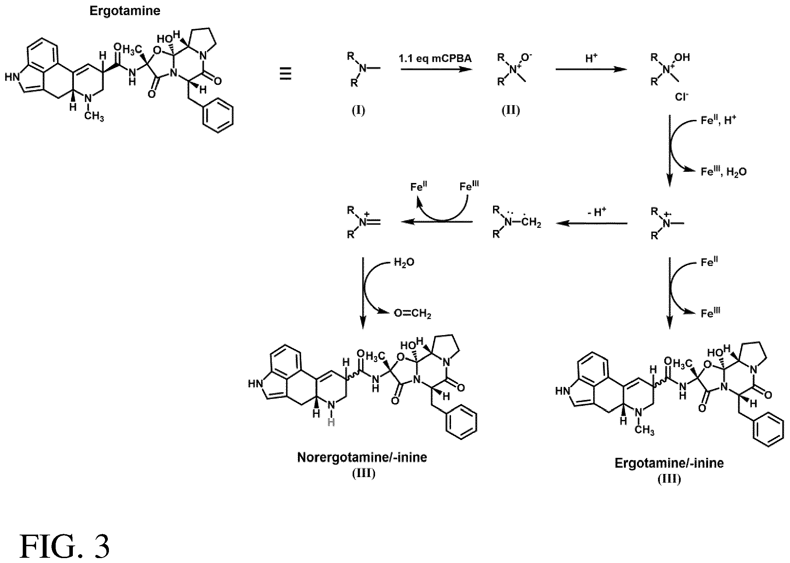

**Fig. 3** shows the reaction mechanism for the *N⁶*-demethylation of ergotamine using an iron catalyzed reaction.

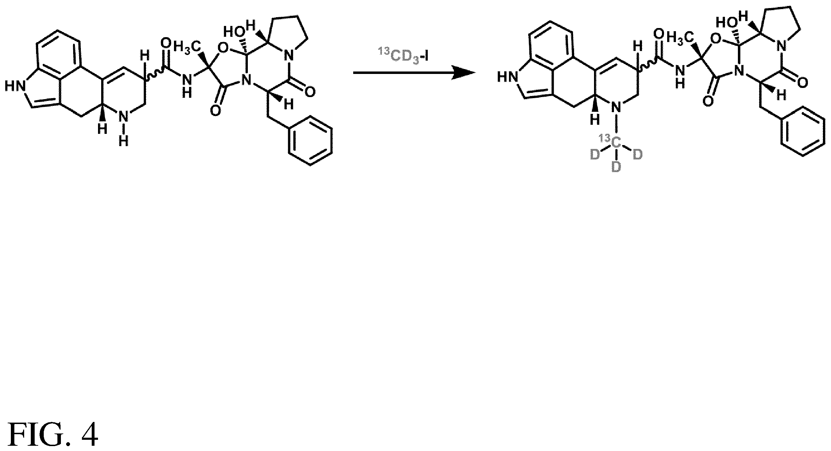

**Fig. 4** shows the reaction of norergotamine/-inine with a suitable isotope of a methylating agent, like e.g. iodomethane (¹³CD₃-I), to ergotamine/-inine-¹³CD₃ which corresponds to the second process step of the suggested synthesis.

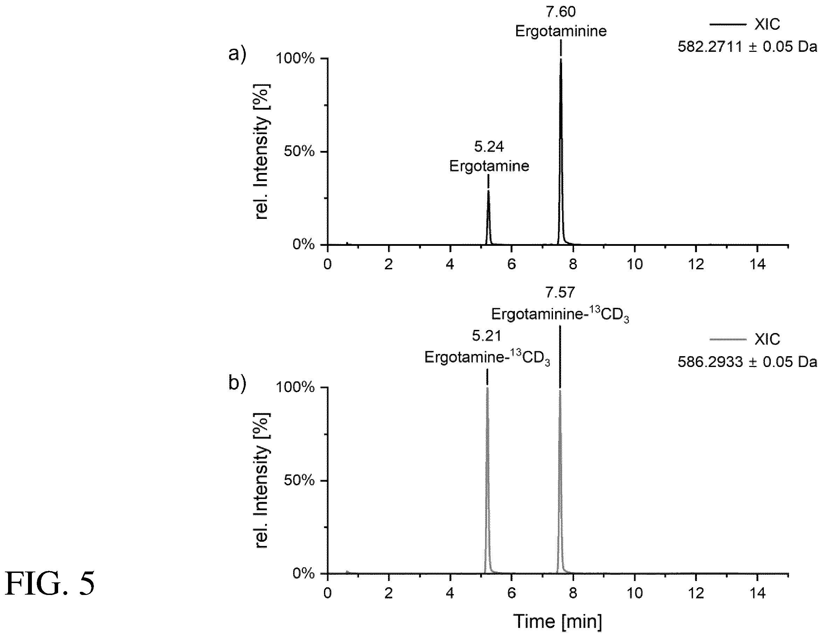

**Fig. 5** shows the extracted ion chromatogram [M+H]⁺ of a) unlabeled ergotamine and ergotaminine (black) **b)** isotopically labeled ergotamine-¹³CD₃ and ergotaminine-¹³CD₃.

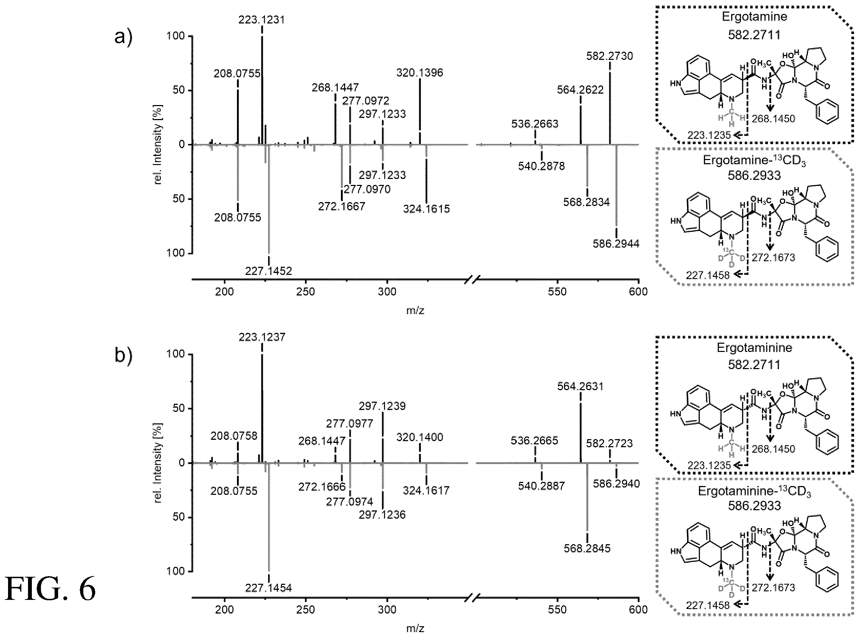

**Fig. 6** shows mass spectra [M+H]⁺ positive electrospray ionization high-resolution tandem MS of **a)** unlabeled ergotamine (black) and isotopically labeled ergotamine-¹³CD₃ (red) **b)** unlabeled ergotaminine (black) and isotopically labeled ergotaminine-¹³CD₃ (red). The structures show the R/S-configurations at the *C⁸*-position, along with the theoretical exact masses of the major fragment ions containing the *N⁶*-atom.

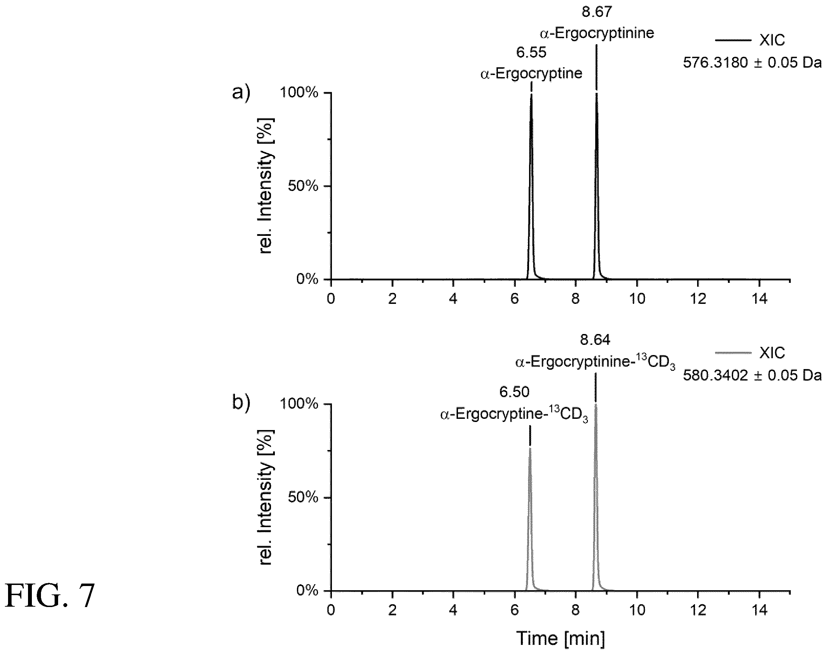

**Fig.** 7 shows the extracted ion chromatogram [M+H]⁺ of **a)** unlabeled α-ergocryptine and α-ergocryptinine (black) **b)** isotopically labeled α-ergocryptine-¹³CD₃ and α-ergocryptinine-¹³CD₃.

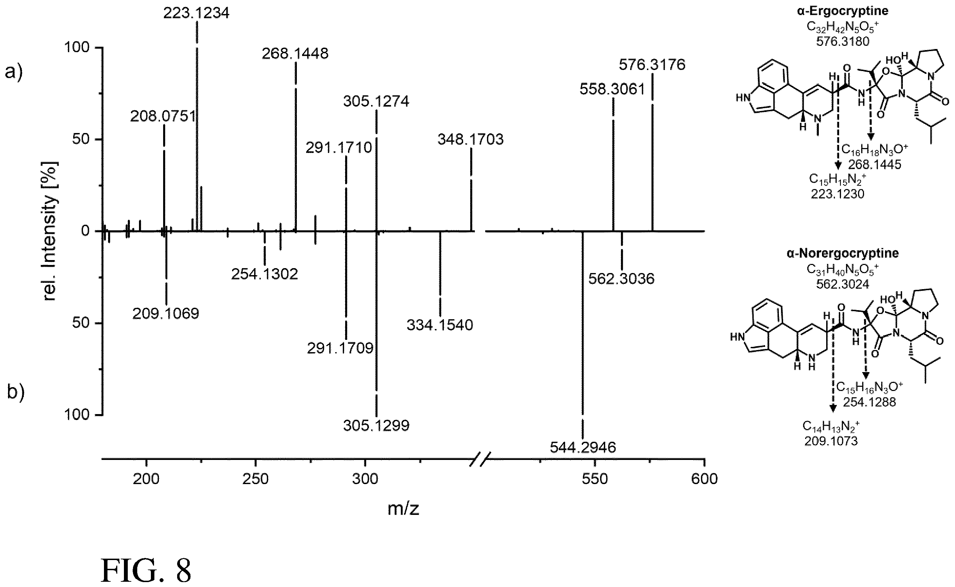

**Fig. 8** Positive electrospray ionization high-resolution tandem mass spectra [M+H]⁺ of **a)** α-ergocryptine and **b)** α-norergocryptine. The structures are displayed on the right-hand side along with the theoretical exact masses for the molecular ion [M+H]⁺ and major fragment ions containing the *N⁶*-atom.

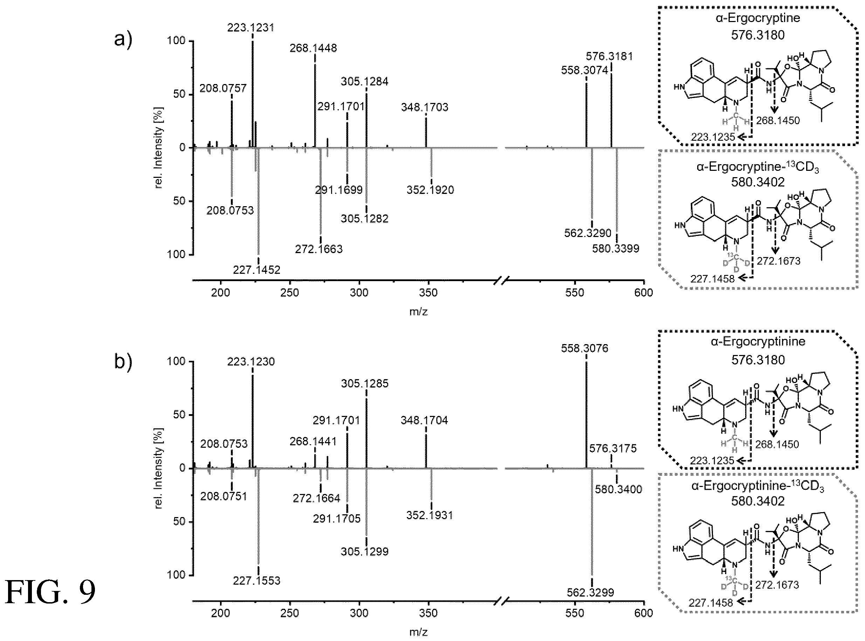

**Fig. 9** shows mass spectra [M+H]⁺ positive electrospray ionization high-resolution tandem MS of **a)** unlabeled α-ergocryptine (black) and isotopically labeled α-ergocryptine-¹³CD₃ (red) **b)** unlabeled α-ergocryptinine (black) and isotopically labeled α-ergocryptinine-¹³CD₃ (red). The structures show the R/S-configurations at the *C⁸*-position, along with the theoretical exact masses of the major fragment ions containing the *N⁶*-atom.

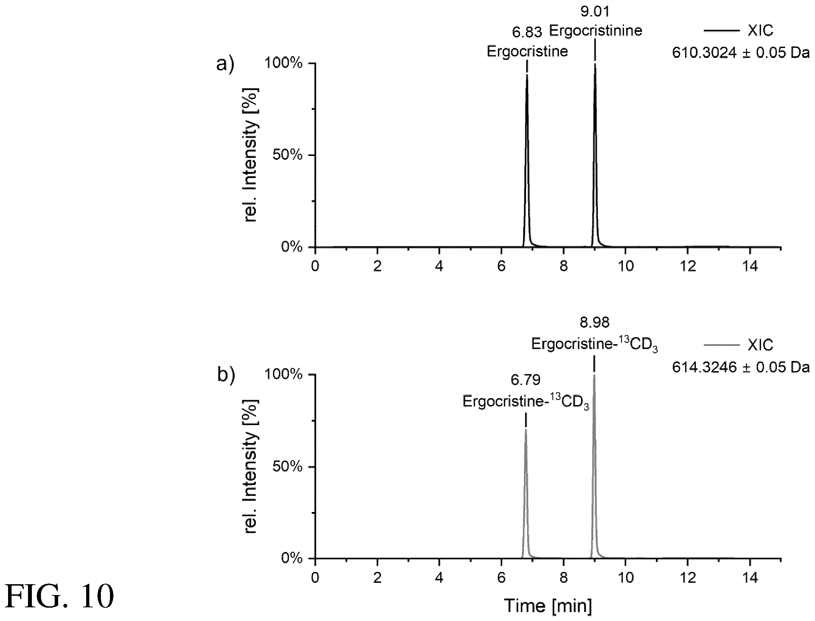

**Fig. 10** shows the extracted ion chromatogram [M+H]⁺ of **a)** unlabeled ergocristine and ergocristinine (black) **b)** isotopically labeled ergocristine-¹³CD₃ and ergocristinine-¹³CD₃.

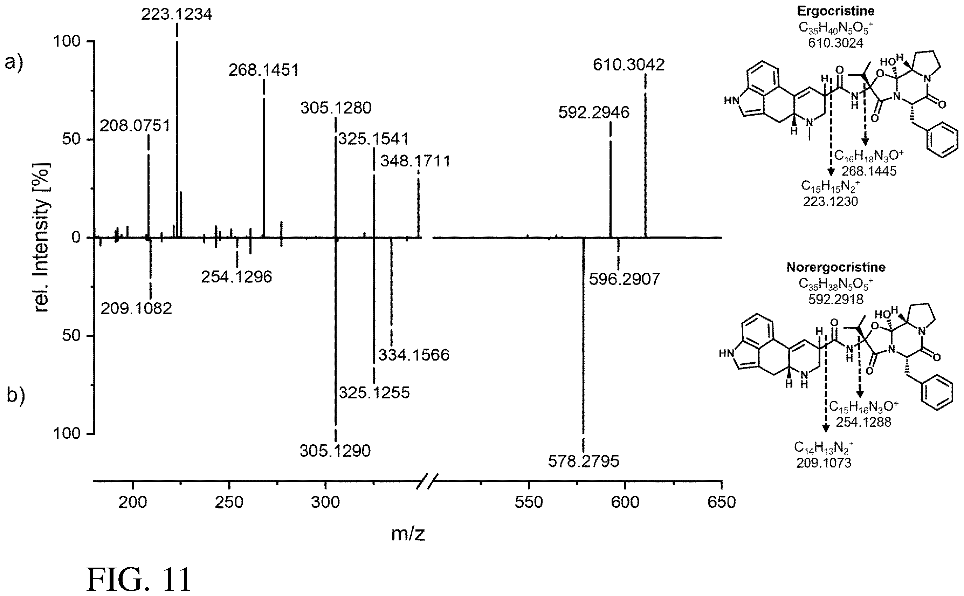

**Fig. 11** Positive electrospray ionization high-resolution tandem mass spectra [M+H]⁺ of **a)** ergocristine and **b)** norergocristine. The structures are displayed on the right-hand side along with the theoretical exact masses for the molecular ion [M+H]⁺ and major fragment ions containing the *N⁶*-atom.

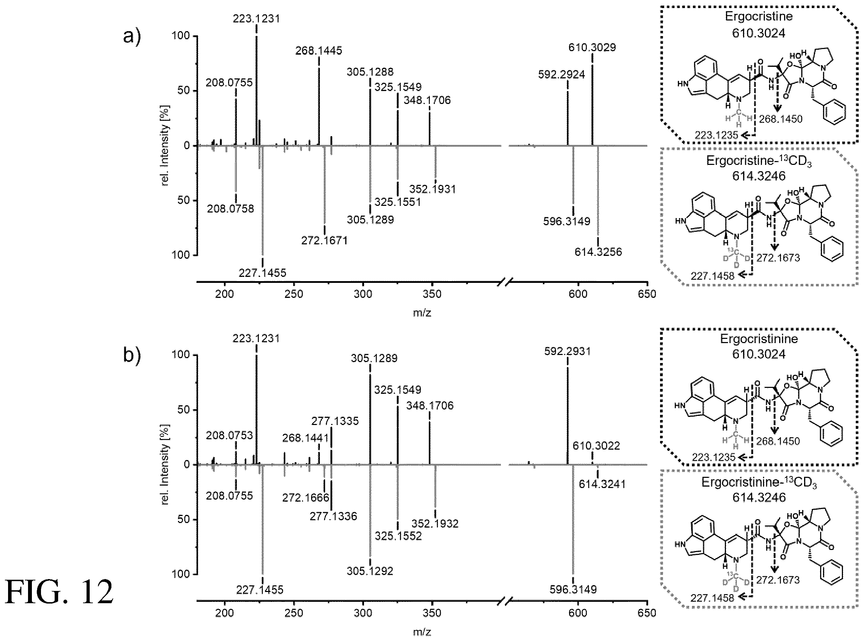

**Fig. 12** shows mass spectra [M+H]⁺ positive electrospray ionization high-resolution tandem MS of **a)** unlabeled ergocristine (black) and isotopically labeled ergocristine -¹³CD₃ (red) **b)** unlabeled ergocristinine (black) and isotopically labeled ergocristinin -¹³CD₃ (red). The structures show the R/S-configurations at the *C⁸*-position, along with the theoretical exact masses of the major fragment ions containing the *N⁶*-atom.

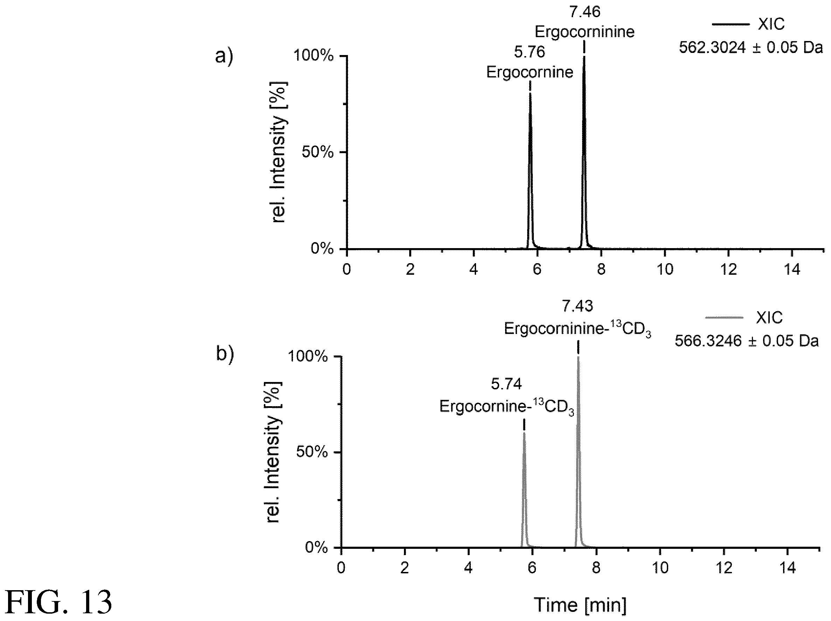

**Fig. 13** shows the extracted ion chromatogram [M+H]⁺ of **a)** unlabeled ergocornine and ergocorninine (black) **b)** isotopically labeled ergocornine-¹³CD₃ and ergocorninine-¹³CD₃.

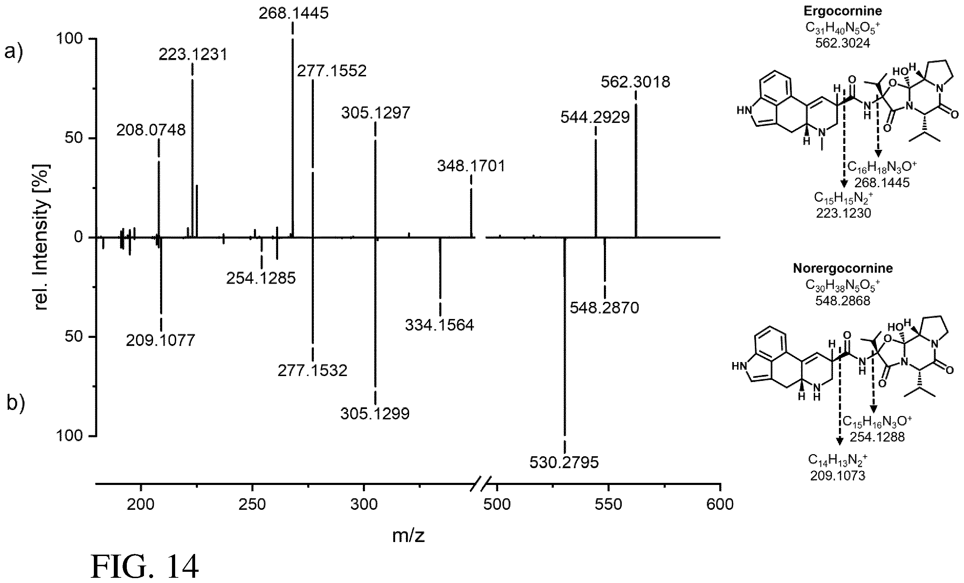

**Fig. 14** Positive electrospray ionization high-resolution tandem mass spectra [M+H]⁺ of **a)** ergocornine and **b)** norergocornine. The structures are displayed on the right-hand side along with the theoretical exact masses for the molecular ion [M+H]⁺ and major fragment ions containing the *N⁶*-atom.

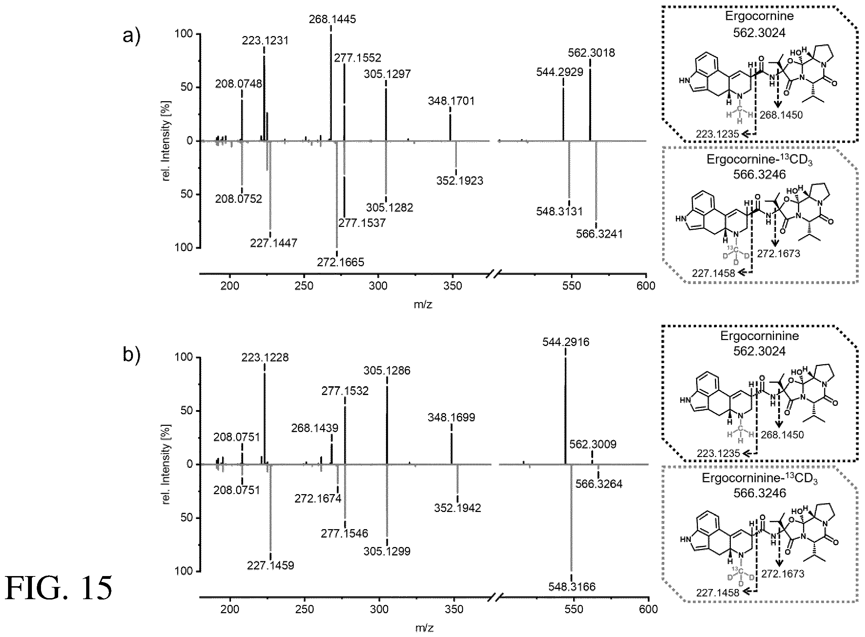

**Fig. 15** shows mass spectra [M+H]⁺ positive electrospray ionization high-resolution tandem MS of **a)** unlabeled ergocornine (black) and isotopically labeled ergocomine-¹³CD₃ (red) **b)** unlabeled ergocorninine (black) and isotopically labeled ergocominine-¹³CD₃ (red). The structures show the R/S-configurations at the *C⁸*-position, along with the theoretical exact masses of the major fragment ions containing the *N⁶*-atom.

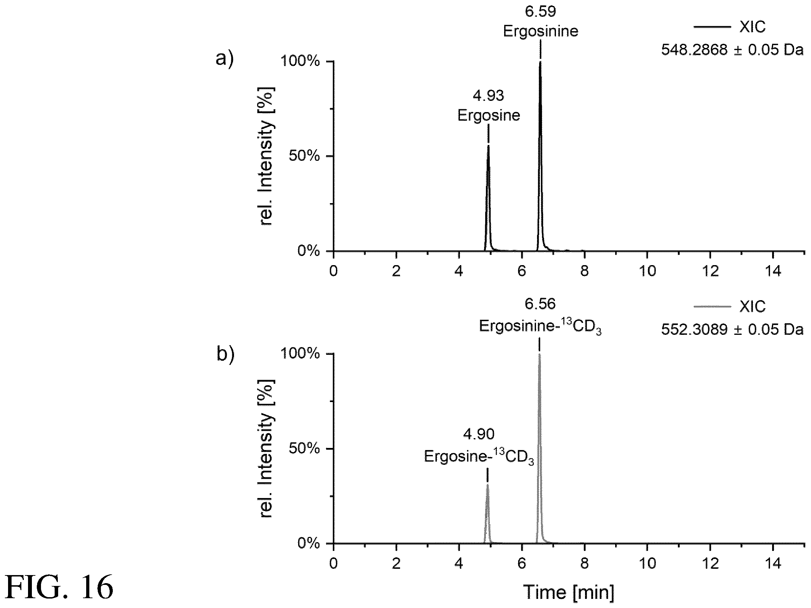

**Fig. 16** shows the extracted ion chromatogram [M+H]⁺ of **a)** unlabeled ergosine and ergosinine (black) **b)** isotopically labeled ergosine-¹³CD₃ and ergosinine-¹³CD₃.

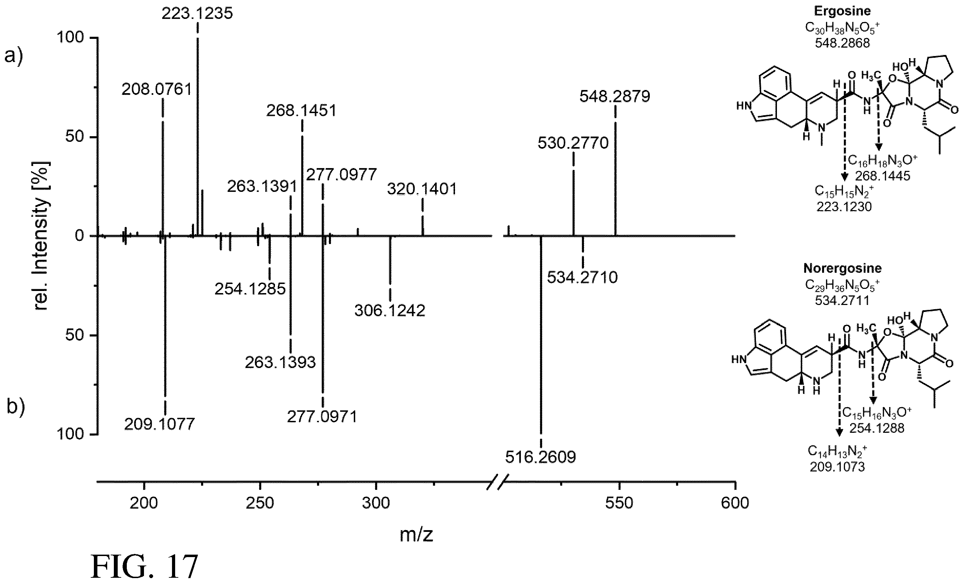

**Fig. 17** Positive electrospray ionization high-resolution tandem mass spectra [M+H]⁺ of **a)** ergosine and **b)** norergosine. The structures are displayed on the right-hand side along with the theoretical exact masses for the molecular ion [M+H]⁺ and major fragment ions containing the *N⁶*-atom.

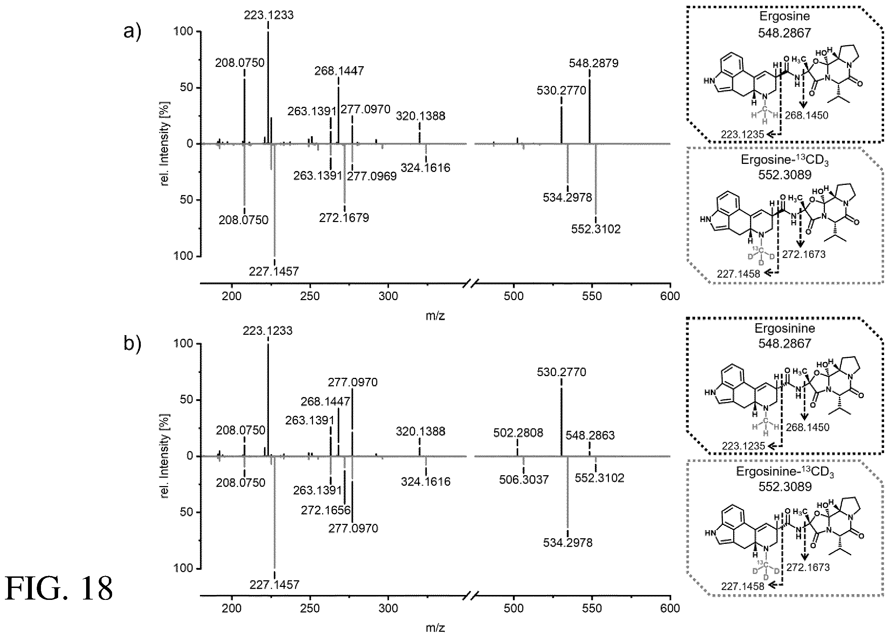

**Fig. 18** shows mass spectra [M+H]⁺ positive electrospray ionization high-resolution tandem MS of **a)** unlabeled ergosine (black) and isotopically labeled ergosine-¹³CD₃ (red) **b)** unlabeled ergosinine (black) and isotopically labeled ergosinine-¹³CD₃ (red). The structures show the R/S-configurations at the *C⁸*-position, along with the theoretical exact masses of the major fragment ions containing the *N⁶*-atom.

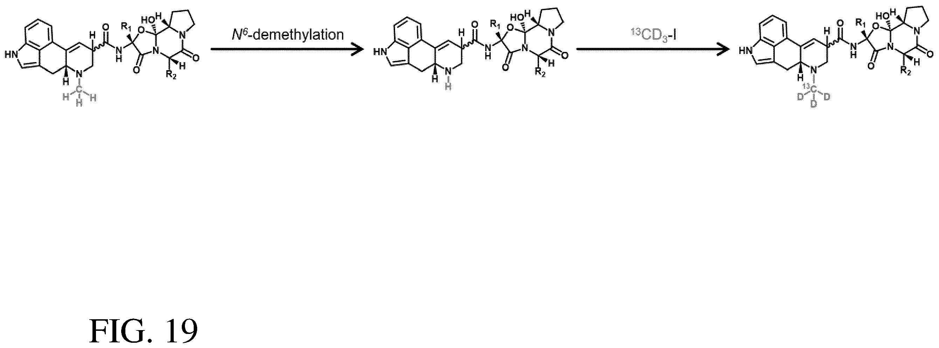

**Fig. 19** shows graphically and generally the demethylation and subsequent remethylation of an ergopeptin (generally shown with residues R₁ and R₂ as indicated in Fig. 1) according to the procedure proposed herein.

In the following detailed description, reference is made to the accompanying figures, which form a part hereof, and which show by way of illustration specific embodiments and features of the invention. It is to be understood that other embodiments may be utilized, and structural or logical changes may be made without departing from the scope of the present invention. The following detailed description, therefore, is not to be taken in a limiting sense, and the scope of the present invention is defined by the claims.

### DETAILED DESCRIPTION

As used in this description (above and below) and claims, the use of the word "a" or "an" when used in conjunction with the term "comprising" in the claims and/or the specification may mean "one", but it is also consistent with the meaning of "one or more", "at least one", and "one or more than one".

As used in this description (above and below) and claims, the use of the word "or" in the claims is used to mean "and/or" unless explicitly indicated to refer to alternatives only or the alternatives are mutually exclusive, although the disclosure supports a definition that refers to only alternatives and "and/or".

As used in this description (above and below) and claims, the words "comprising" (and any form of comprising, such as "comprise" and "comprises"), "having" (and any form of having, such as "have" and "has"), "including" (and any form of including, such as "includes" and "include"), "containing" (and any form of containing, such as "contains" and "contain") or "encompassing" (and any form of encompassing, such as "encompass" and " encompasses") are inclusive or open-ended and do not exclude additional, unrecited elements or method steps.

The expressions Fe(II) ,Fe(III), Fe^{II} and Fe^{III} are used here as synonyms for the expressions Fe(2+) and Fe(3+) respectively. Since both ions occur in solution and can remain in it regardless of a change in charge, the terms Fe(II/III) and Fe(2+/3+) are also used as synonyms. As indicated below, Fe(0), i.e. elemental iron, e.g. as fine iron powder, can also be used as a catalyst for the proposed synthesis as it is oxidized in situ to the corresponding catalytic active Fe^{II} species.

According to one embodiment, a synthesis is suggested comprising chemically modifying unlabeled ergot alkaloids, particularly ergot alkaloids, with a stable carbon isotope in order to obtain the labeled ergot alkaloids. In particular, the synthesis comprises firstly demethylating the ergot alkaloid and secondly remethylating the ergot alkaloid with an isotopically labeled methyl group. The demethylation, resulting in the corresponding norergot alkaloid, may be followed by purification, e.g. using chromatography. The thus purified norergot alkaloid is then, in the second step, remethylated using an isotopically labeled methyl group. The isotopically labeled methyl group comprises any of ¹¹C, ¹²C, ¹³C, ¹⁴C, ¹H, ²H, i.e. D (deuterium), and ³H, i.e. tritium.

As ¹¹C has a half-life of only about 20.3 minutes and is radioactive, its usability in an internal standard is limited. The applicability of ¹¹C, ¹⁴C, and tritium due to their radioactivity is also limited. However, advantageously, the suggested process of synthesis is easy to perform and has a yield as high as 9-29%. As indicated further below for ergotamine-¹³CD₃ and ergotaminine-¹³CD₃ (See **Figs. 2 - 6****)** yields of about 15%, for ergocornine-¹³CD₃ and ergocominine-¹³CD₃ (See **Figs. 13 - 15****)** yields of about 20% and for ergocristine-¹³CD₃ and ergocristinine-¹³CD₃ (See **Figs. 10 - 12****)** yields of about 14 % were obtained. Ergosine-¹³CD₃ and ergosinine-¹³CD₃ (See **Figs. 16 - 18****)** was diluted in acetonitrile/water after purification with HPLC and a yield of about 9% was determined by HPLC-MS/MS. As indicated below, this yield could even be increased to 29 % for ergocryptine-¹³CD₃ and ergocryptinine-¹³CD₃ (See. **Figs. 7-9****).** Further, due to the simple reaction scheme used, the suggested synthesis process ( See **Fig. 19****)** does not result in as many side products as previously known techniques do, and is therefore less expensive. A purification of the finally obtained product, i.e. of the isotopically labeled ergot alkaloid is typically not necessary, but may optionally be performed. Appropriate purification techniques comprise liquid chromatography, e.g. HPLC. Suitable chromatography columns or cartridges and/or suitable chromatographic carrier materials are known to the skilled person.

According to an embodiment, the demethylating is carried out in the presence of an iron-containing catalyst, preferably the iron-containing catalyst comprises any of an Fe(0), Fe(2+), Fe(3+), or a complex thereof, such as a porphyrin iron (2+/3+) complex, e.g., an iron porphyrinate.

Advantageously, elementary iron and iron (II) and iron (III) ions are easily available. Typically, iron (II/III) - complexes are water soluble, e.g. porphyrin complexes such as bio-inspired and/or biomimetic iron (II) and iron (III) complexes are water soluble.

According to an embodiment, the iron porphyrinate is selected from: iron(2+/3+) tetrabenzotetraazaporphyrin, iron(2+/3+) tetraazaporphyrin, iron(2+/3+) trabenzoporphyrin and/or iron(2+/3+) porphyrin, i.e. a bio-inspired cytochrome-P450-derivative shown below.

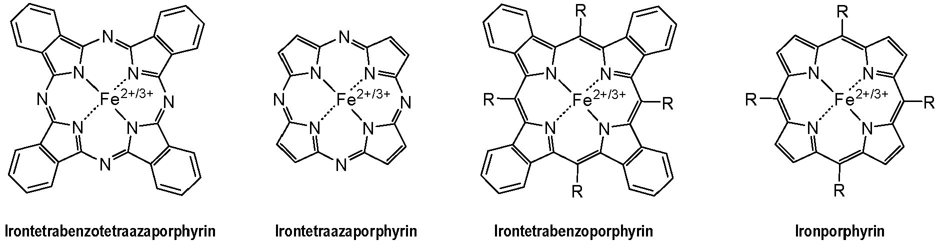

According to an embodiment, in the corresponding formulae above the residues R are identical for all four indicated positions of a molecule and are selected from: 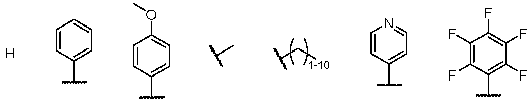 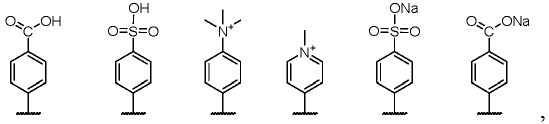 preferably from: 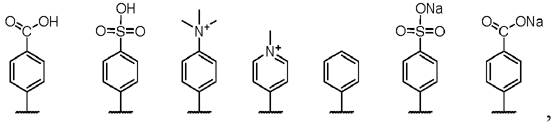 more preferably from:

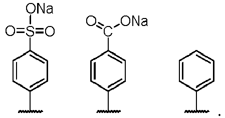

Advantageously, said residues R provide favorable solubility of the catalysts in corresponding solutes, e.g., the acids and the corresponding salts of alkali metal (e.g. sodium, potassium, etc.) in polar solvents and/or aqueous solutions. Additionally, the heteroatoms e.g., oxygen increase the bonding points for hydrogen bond between the catalyst and the ergot alkaloid-*N⁶*-oxide, further reducing the diffusion rate of the catalyst. The permanently charged 4-trimethylammoniophenyl- and 4-methylpyridinium-residue improve solubility in polar solvents and/or aqueous solutions. The phenyl residue provides favorable solubility in more hydrophobic solution e.g., dichlormethane and/or toluene.

According to the invention, a *N*-demethylation reaction (Fig. 3) is applied to the initial (i.e. starting) ergot alkaloid, which is demethylated and partially converted to the corresponding N⁶-demethylated norergot analog, while a remainder is reacting to the original ergot alkaloid. As apparent, the indicated reaction scheme is universal for all ergot alkaloids discussed herein, even if it is illustrated there for ergotamine/-inine.

In particular, Fe(II) in a bioinspired porphyrin complex (or a derivative thereof) is added to the reaction solution after the N⁶-oxidation of the ergot alkaloid (e.g. with mCPBA). The iron(II)-complex is replacing the addition of an iron salt as it is acting as catalyst. During the reaction, the iron(II) center of the porphyrin is oxidized to iron(III) under the formation of an aminium radical cation (Fig.3). While still bound to iron, the cleavage of an α-proton and electron reorganization results in the formation of a more stable carbon centered radical. The carbon-centered radical is oxidized by iron(III) to produce an iminium ion, which subsequently hydrolyzes to yield the N-demethylated product. The primary byproduct is the parent tertiary amine which is recycled, so to speak, in the described synthesis. One possible explanation for the formation of the primary byproduct is that the intermediate aminium radical cation dissociates from the oxidized iron complex and undergoes further reduction by iron(II). According to the invention, the yield of N-demethylation can therefore be significantly increased by using Fe(II/III) complexes, such as Fe(II/III) derivatives of porphyrin, preferably by one of the above indicated ones. Without being bound by theory, the reaction mechanism of the proposed synthesis can explain the technical effect obtained according to this embodiment of the invention: Since low molecular weight components have a significantly higher diffusion rate, the yield of the N-demethylated ergot alkaloid and therefore also the labeled ergot alkaloid is further increased by using high molecular weight complexes, such as porphyrin complexes of iron, compared to catalytically active compounds having a lower molecular weight, e.g. iron ions.

According to an embodiment, the isotope-labeled methylating reagent is selected from the group consisting of ¹²C and ¹³C analogs of the following substances, wherein hydrogen H is substituted by deuterium D:
acetic acid; iodomethane or methyl iodide and its derivatives, such as e.g. bromomethane dimethyl carbonate; dimethyl sulfate; dimethyl sulfite; formaldehyde; formic acid;
dimethyl sulfate; dimethyl sulfoxide; dimethyl carbonate; methanol;
methyl 4-nitrobenzenesulfonate; methyl-fluorosulfonate; methyl- trifluoromethane sulfonate; *p*-toluene sulfonic acid methylester and trimethyloxonium tetrafluoroborate.

Advantageously, the listed substances are known as effective methylating agents. With regard to the suggested isotopes, it is evident that the greater the difference in mass between the natural and isotope-enriched ergot alkaloid, the better the corresponding substance can be quantified by mass spectrometry. Therefore, ¹³C analogs are preferable to ¹²C analogs. The use of deuterium (D), e.g. in a methyl group instead of hydrogen (H) alone leads to a mass shift Δm of 3. In addition, the specified substances are not radioactive and can therefore be used without special precautions.

According to an embodiment, the ergot alkaloid is selected from any of the diastereoisomer pairs of ergocornine/ergocorninine; ergometrine/ergometrinine, ergocristine/ergocristinine, ergotamine/ergotaminine, ergosine/ergosinine, α-ergocryptine/ α-ergocryptinine, and β-ergocryptine/β-ergocryptinine. Thus, in other words, said group consists of a *5R*, *8R* configuration and a *5R, 8S* configuration of said ergot alkaloids. Herein *5R, 8R* corresponds to the *-ine* form (e.g. ergotamine, ergocryptine, ergocristine, etc.) and the *5R, 8S* corresponds to the *-inine* form (ergotaminine, ergocryptinine, ergocristinine, etc.). The number of members of this group is thus 14. In this context, we disregard the fact that the relevant Commission Regulation (EU) 2023/915 only covers the maximum content of ergot alkaloids of the α- and β-forms of ergocryptine/ergocryptinine as a sum, i.e. ergocryptine in general, and hence relates to only 12 substances are monitored in total: "*Lowerbound sum of ergocornine*/ *ergocorninine; ergocristine*/*ergocristinine; ergocryptine*/*ergocryptinine (α- and β-form); ergometrine*/*ergometrinine; ergosine*/*ergosinine; ergotamine*/*ergotaminine.* "

Advantageously, the mentioned substances are the ergot alkaloids which are to be monitored according to the corresponding regulations.

According to an embodiment, the ergot alkaloid comprises a *5R, 8R* configuration and/or a *5R, 8S* configuration of: ergotamine, ergocryptine, particularly α-ergocryptine, ergocornine, ergosine and/or ergocristine.

The isomers mentioned can transform into each other depending on the external conditions. The ergot alkaloids, which are very toxic in their *5R,8R* configuration, are responsible for ergotism, which occurs as a result of long-term ergot poisoning. Symptoms of ergot poisoning are constriction of the blood vessels and circulatory disorders, for example in the heart or limbs. In the most severe cases, limbs such as fingers or toes can die. Therefore, both the *5R,8R* and *5R,8S* forms of ergot alkaloids must be precisely quantified in cereals and cereal products using the labelled ergot alkaloids as synthesized according to the present disclosure.

According to an embodiment, the provided ergot alkaloid, i.e. the ergot alkaloid which is used as educt in the disclosed synthesis is a biosynthetically generated ergot alkaloid, in particular a native, i.e. a naturally occurring, ergot alkaloid which had been extracted from a sclerotium of *Claviceps spec.* and/or which had been isolated from a cell culture of a microorganism.

Advantageously, common food and feed contaminants can be reliably detected and quantified and thus monitored in accordance with the applicable regulations for grain, cereals and related food and feed products. In particular, Appendix C, page 29 of the mentioned EN 17425 states: "The performance of the method may be improved by using an isotope-labeled internal standard, but this was not available at the time of method validation."

According to an embodiment, a mass shift Δm of the synthesized ergot alkaloid, which can be used in mass spectrometry as internal standard in comparison to the natural ergot alkaloid is in a range of: 3-6, i.e. 3 ≤ Δm ≤ 6, particularly in the range of 4-5.

Advantageously, the indicated mass difference Δm is large enough to be distinguished from the corresponding unlabeled ergot alkaloid and fragments thereof.

According to an embodiment a method for detecting an ergot alkaloid in a sample, comprising determining a N⁶-isotope-labeled ergot alkaloid is suggested, wherein determining encompasses a mass spectrometry of the sample, of an extract of the sample, or of an aliquot of the sample. For instance, said method is selected from:
- a mass spectrometric analysis of the sample, such as HPLC-MS or tandem mass spectrometry (MS/MS) analysis, using a N⁶-isotope-labeled ergot alkaloid as an internal standard, and
- a stable isotope dilution (SID) methodology, as described, e.g., by Ciccimaro E. and Blair I. A. (2010) "Stable-isotope dilution LC-MS for quantitative biomarker analysis" in Bioanalysis 2(2), 311-341; https://doi.org/10.4155/bio.09.185.

Anyway, i.e. in both cases suggested above, the N⁶-isotope-labeled ergot alkaloid is synthesized according to the disclosed herein synthesis. The "stable isotope dilution" / SID methodology is also known as SIDA, i.e. stable isotope dilution analysis, which is based on a liquid chromatography and mass spectrometry.

Advantageously, the molecular ion and fragments thereof appearing in a mass spectrum can securely be identified and differentiated from the native ergot alkaloids.

According to an embodiment a N⁶-isotope-labeled ergot alkaloid is suggested, wherein the ergot alkaloid is selected from the group consisting of:
a *5R, 8R* configuration of ergocornine, a *5R, 8S* configuration of ergocornine,
a *5R, 8R* configuration of ergometrine, a *5R, 8S* configuration of ergometrine,
a *5R, 8R* configuration of ergocristine , a *5R, 8S* configuration of ergocristine ,
a *5R, 8R* configuration of ergotamine , a *5R, 8S* configuration of ergotamine,
a *5R, 8R* configuration of ergosine, a *5R, 8S* configuration of ergosine,
a *5R, 8R* configuration of α-ergocryptine, a *5R, 8S* configuration of α-ergocryptine,
a *5R, 8R* configuration of β-ergocryptine, and a *5R, 8S* configuration of β-ergocryptine.

Advantageously, the N⁶-isotope-labeled ergot alkaloid can be used in a mass spectrometry analysis of a sample which is potentially contaminated by an ergot alkaloid, wherein the ergot alkaloid is selected from the indicated group. Both mentioned techniques HPLC-MS/MS and SID, comprising MS can be applied.

According to an embodiment the mentioned N⁶-isotope-labeled ergot alkaloid comprises any of ¹¹C, ¹²C, ¹³C, ¹⁴C, ¹H (hydrogen), ²H (deuterium), and ³H (tritium), preferably the stable isotopes ¹³C and ²H (deuterium).

Although non-radioactive derivatives are preferred for obvious reasons, obtainable mass shifts Δm are also achievable with long-lasting radionuclides (¹⁴C, ³H). The significant mass shift Δm of the labeled and unlabeled ergot alkaloid Δm ≥ 3 ensure safe identification of corresponding fragments by mass spectrometry.

According to an embodiment, a kit for detection of a mycotoxin by using a mass spectrometry is suggested. Said kit comprises at least one N⁶-isotope-labeled ergot alkaloid according to any of the embodiments described above and further below, wherein the ergocornine, the ergometrine, the ergocristine, the ergotamine, the ergosine, the α-ergocryptine, and the β-ergocryptine, whether in its *5R, 8R* configuration or in its *5R, 8S* configuration, is synthesized as disclosed herein. Thus, the N⁶-isotope-isotope-labeled ergocornine, ergometrine, ergocristine, ergotamine, ergosine, α-ergocryptine, and/or β-ergocryptine is first synthesized by demethylation of the corresponding native ergot alkaloid and its subsequent remethylation, preferably using non-radioactive carbon and hydrogen isotopes.

Advantageously, this kit can be used for unambiguous identification and thus for monitoring practically important ergot alkaloids.

According to an embodiment the kit comprises at least one vial (ampoule, container etc.) containing at least one of the N⁶-isotope-labeled ergot alkaloids in a dry or otherwise stabilized form, , e.g. in a lyophilized form or as a solution or as a dispersion, wherein a quantity of the dry or otherwise stabilized isotopomer ergot alkaloid in the vial is adapted to allow generating a defined concentration of said ergot alkaloid by simply dissolving and/or diluting it with a defined liquid volume.

Using the defined volume of liquid for reconstitution, the dry or otherwise stabilized ergot alkaloid, i.e. a defined amount thereof, contained in the vial can be directly transferred into a standard solution containing a defined concentration of the N⁶ isotope-labeled ergot alkaloid to be used in a mass spectrometry, e.g. as an internal standard. Advantageously, a person entrusted with the determination of concentrations of the ergot alkaloids to be detected in a sample can prepare one or more defined standard solutions comparatively easily. The standard solutions, which contain at least one isotope-labeled ergot alkaloid and which are advantageously prepared immediately prior to the analysis of the respective sample material, can be added to the sample in a known quantity. From the measurement results of the sample "spiked" in this way, the current concentration of one or all of the ergot alkaloids to be monitored can be determined with less error in accordance with the applicable guidelines.

Further, the isotopically labeled ergot alkaloids of the kit are stable and can thus be used in monitoring of ergot alkaloid concentrations in food and feed. Advantageously, the described synthesis and accordingly the described kit allows to our knowledge for the first time the correct and retraceable standardized implementation of the legally binding regulations according to Commission Regulation (EU) 2023/915 and EN 17425 (2021) and of other corresponding national regulations.

According to an embodiment using an N⁶-isotope-labeled ergot alkaloid in a mass spectrometry is suggested, wherein the N⁶-isotope-labeled ergot alkaloid is produced by the two-step synthesis as disclosed herein.

Advantages are the already indicated ones: low costs, high yield, unambiguous identification, secure handling.

The above embodiments can be combined with each other.

As to the object of the present application, **Fig. 1** shows the 6 most important ergot alkaloids and their C⁸ stereoisomers, i.e. 14 isomers in total. As illustrated, their structure is based on lysergic acid, shown in the middle of **Fig. 1****.** Resulting lysergic acid amides can be divided into two distinct groups: simple lysergic acid derivatives, such as ergometrine, and the group of ergopeptines with a tricyclic peptide ring. The corresponding stereoisomers are formed by isomerization at the *C⁸*-carbon atom of the lysergic acid moiety. The compounds comprising a *C⁸*-(*R*) configuration are referred to as ergopeptines (e.g., ergotamine) and the compounds comprising the *C⁸*-(*S*) configuration as ergopeptinines (e.g., ergotaminine).

The suggested approach of demethylating and subsequent remethylating is based on unlabeled native ergot alkaloids which can easily be extracted from native material and/or can be commercially obtained at significantly lower prices than their synthetic analogues. However, a synthetically generated alkaloid can also be labeled using the suggested synthesis process.

**Fig. 2** shows the relevant masses of the fragments [M+H]⁺ of ergotamine **(a)** and norergotamine **(b)** obtained by high-resolution tandem mass spectrometry with positive electrospray ionization. As evident from the fragmentation schemes on the right, the demethylated structure (norergotamine) yields fragments with correspondingly lower masses, e.g. 209.1077 vs. 223.1239 and 254.1294 vs. 268.1440. The smaller fragments correspond to the *N⁶*-demethylated lysergic acid moiety, confirming the structure of the target norergotamine.

**Fig. 3** illustrates the proposed reaction scheme used according to the suggested synthesis process. Starting from an ergot alkaloid (here ergotamine) the tertiary amine (I) at N⁶ is converted into the corresponding N-oxide (II) using an organic or inorganic peroxide.

In detail, the tertiary amine e.g. here ergotamine (I) is oxidized with a peroxide (e.g., hydrogen peroxide, mCPBA or peroxymonosulfuric acid) into an amine *N*-oxide (II). As amine N-oxides are weak bases, the addition of an acid (e.g., hydrochloric acid or sulfuric acid) results in the protonation of the oxygen, forming a cationic hydroxylamine (R₃-N⁺-OH). The addition of an Fe(II) and/or Fe(III) in the form of a salt and/or complex or iron powder (Fe0) catalyzes a rearrangement reaction in which the cleavage of the formed iminium cation results in the formation of the N-demethylated product (III). The main by-product is the originally used tertiary amine (I) and can therefore be recovered and reused. As apparent, the cyclic peptide during the synthesis steps remains untouched. The two-step synthesis as suggested is therefore applicable to all ergot alkaloids mentioned above.

Although the synthesis proposed here may be described in detail above, it is clearly characterized by its simplicity and the advantageous recycling of the starting product (ergot alkaloid). The ergot alkaloid in question is used directly to obtain the corresponding isotopomer. The peptide of the ergot amine remains unmodified as has been demonstrated for other ergot alkaloids as well.

In contrast, the synthesis described in Braun B. et al. (2017) is based on the isotopic modification of lysergic acid and the subsequent coupling of the modified lysergic acid with (S)-alaniol to obtain the desired isotopic ergometrine. This synthesis according to Braun B. et al. (2017) comprises a total of 6 basic steps and would be even more complicated, if at all possible, for the other ergot alkaloids, which comprise cyclic peptides instead of the 1-hydroxypropan-2-yl group. In contrast, the synthesis strategy disclosed here comprises only 2 basic steps: demethylation and remethylation of the corresponding ergot alkaloid. Different to Braun B. et al. (2017) we use the complete ergot alkaloid, not its constituent lysergic acid. Thus, as exemplified, the two-step synthesis is suitable for all ergot alkaloids. As mentioned above, the first and/or second basic step may optionally be followed by a purification, e.g. by chromatographic enrichment of the main product. It is obvious that the synthesis scheme described by Brown B. et al. (2017) for the simplest of the ergot alkaloids is significantly more complex, thus more expensive and, above all, significantly less productive.

**Fig. 4** illustrates the second reaction step of the suggested process of synthesizing the ¹³CD₃ isotopomers of ergotamine/ergotaminine.

**Fig. 5** shows extracted ion chromatograms of unlabeled ergotamine (top) and isotopically labeled ergotamine-¹³CD₃ (bottom). As evident, the peaks of both labeled and unlabeled ergotamine and ergotaminine in the extracted ion chromatograms (XIC) align perfectly. Thus, both native and labeled alkaloids having the same retention time behave identically. Therefore, the synthesized isotopomers can be used to spike an unknown sample in order to identify and quantify the corresponding mycotoxin therein.

The lines in the mass spectra of **Figs. 6(a)** and **6(b)** represent the R/S-configurations at the *C⁸*-position, along with the theoretical exact masses of the major fragment ions containing the *N⁶*-atom of the unlabeled substances and corresponding synthesized ISTD of ergotamine and ergotaminine, respectively. The high resolution (HR)-MS/MS spectra for both epimers are identical for the labeled and unlabeled substances. Notably, a distinct mass shift of Δm=4 Da is observed in the isotopically labeled molecules, especially within the fragment ions containing the labeled *N⁶*-atom.

**Figs. 7-9** relate as indicated to α-ergocryptine/-inine, the **Figs. 10-12** relate to ergocristine/-inine, the **Figs. 13-15** relate to ergocornine/-inine, the **Figs. 16-18** relate to ergosine/-inine and correspond to the previously discussed measurement results of ergotamine/-inine illustrated by **Figs. 4-6****.**

As indicated, the processes of partial (starting from labeled lysergic acid or labeled peptides) or total synthesis are time consuming and complex, resulting in low overall yields and high production costs. The approach suggested according to the present invention is to begin with the unlabeled native ergot alkaloid followed by subsequent chemical modifications to obtain the labeled ergot alkaloid. It is important to consider the specific position to be modified and select a group that is large enough to accommodate a mass shift (Δm) of at least 3 Da between the native and labeled ergot alkaloid. With a Δm of 4 Da compared to 3 Da or less, the isotopic distribution of the native ergot alkaloid has less influence on the signal of a corresponding fragment of the isotopically labeled species. As this directly affects quantification by isotope dilution analysis, the so-called SID or SIDA, it was the object of the present invention to maximize the mass shift Δm between the native and the isotopic labeled ergot alkaloid. Increasing the mass shift beyond 4 Da would further decrease the influence of the isotopic pattern of the native ergot alkaloid on the signal of the isotopically labeled ergot alkaloid. However, it would also complicate the choice of a specific position to be modified.

As all the important ergot alkaloids share the lysergic acid structure methylated at the *N⁶*-position, we focused on the feasibility of a N-demethylation at the *N⁶*-position of native ergot alkaloids and the subsequent remethylation with an isotopically labeled methyl group.

The demethylation of the ergot alkaloids may be reached by different methods, e.g. using electrochemical methods (See Jahn S. et al. (2011) Investigation of the biotransformation pathway of verapamil using electrochemistry/liquid chromatography/ mass spectrometry - a comparative study with liver cell microsomes. J Chromatography A 1218 (9210-9220); Torres S. et al. (2014) Rapid Synthesis of Pharmaceutical Oxidation Products Using Electrochemistry: A Systematic Study of N-Dealkylation Reactions of Fesoterodine Using a Commercially Available Synthesis Cell. Organic Process Research & Development 19(1596-1603); and Najmi A.A. et al. (2021) TEMPO-Mediated Electrochemical N-demethylation of Opiate Alkaloids. Chem Electro Chem 8 (2590-2596).

However, as outlined further below, due to insufficient yield, unwanted by-products, and problems with scale-up, we later on used a chemical demethylation according to the practical examples described below.

Surprisingly, the synthesis method proposed here, comprising merely two basic process steps: a demethylation at N⁶ of the ergot alkaloid and a subsequent remethylation at the N⁶, for example using ¹³CD₃I (methyl iodide), can be applied to all relevant ergot alkaloids mentioned above and below, whereby isotope-labeled analogs of the practically important ergot alkaloids become accessible.

To summarize, the initially indicated objective of the present invention could surprisingly be reached by applying the reaction shown in **Fig. 3** as disclosed herein. Thus, an effective labeling method comprising merely two basic synthesis steps is provided. The suggested synthesis is simple and can be used universally for generating isotopically labeled equivalents of all ergot alkaloids which are required as standards for ergot alkaloid monitoring according to the required regulations. The merely two steps of the suggested labeling process result *per-se* in a higher yield of the labeled ergot alkaloids than a multistep procedure could provide. Further, due to the recycling of the respectively used educt, i.e. re-use of the unmodified ergot alkaloids within the synthesis process, the yield of the labeled substance is further enhanced. The suggested synthesis process is loss-free as nothing is wasted by unwanted by-products. Therefore, the disclosed process of synthesizing avoids the disadvantages of previously known methods and provides standard substances for mass spectrometry at lower costs.

### PRACTICAL EXAMPLES

### Ergotamine

As a major representative of the ergopeptine group ergotamine has been used in a first demethylation approach. Subsequent remethylation at the *N⁶*-atom with an isotopically labeled methylation reagent should yield ergotamine/-inine-¹³CD₃ which than could be used as internal standard during HPLC-MS/MS analysis.

### Electrochemical N-demethylation

Various working and auxiliary electrodes, electrolytes, and solvents were tested for N-demethylation **(Table 1).** The reaction solutions were pumped through an electrochemical cell and the voltage was ramped up continuously from 0 to 1.2 V. The reaction products were analyzed directly using mass spectrometry and via HPLC-MS.

**Table 1**

| Overview of the different working- and auxiliary electrodes (GC: glassy carbon; BDD: boron doped diamond; Pt: platinum; PEEK: polyether ether ketone) and concentration of electrolytes/additives used for the electrochemical reactions. The concentration of ergotamine was 10 µM in water/acetonitrile or water/methanol in different ratios (v % / v %) for each experiment. | | | |
|---|---|---|---|
| **Working-/auxiliary electrode** | **Electrolyte** | **10 µM Ergotamine in Water / Acetonitrile or Water / Methanol (v % / v %)** | **N-demethylated product detected** |
| GC, BDD and Pt/ PEEK | 10 mM NH₄OOCH₃ | 0 / 100 ; 20 / 80 and 50/50 | ✔ (only GC) |
| | 10 mM formic acid | 0 / 100 ; 20 / 80 and 50 / 50 | × |
| | 10 mM (NH₄)₂CO₃ | 20 / 80 and 50/50 | ✔ (only GC) |

When the reaction was carried out in methanol, only a methoxy adduct of ergotamine was detected under all tested conditions. Therefore, we decided to test acetonitrile instead of methanol. Using ammonium acetate or ammonium carbonate as an electrolyte with a glassy carbon (GC) electrode at a voltage of 0.6 V, we were able to detect a product with an appropriate nominal mass-to-charge ratio (*m*/*z*) of 568. This *m*/*z* corresponds to the theoretical nominal mass of the *N⁶*-demethylated ergotamine (norergotamine). The use of formic acid as an electrolyte did not result in the formation of a product with *m*/*z* 568. A sample of the reaction solution was collected and analyzed using high-resolution tandem mass spectrometry (HR-MS/MS). **Fig. 2** displays the tandem mass spectra of unreacted ergotamine and the product (norergotamine) with *m*/*z* 568 and the corresponding fragment with *m*/*z* 209.

In particular, both spectra of ergotamine (a) and norergotamine (b) shown in **Fig. 2** exhibit a similar fragmentation pattern, however the different intensities of certain fragment ions can be attributed to the lower energy required for the fragmentation of norergotamine. Fragment ions with a Δ*m*/*z* of 14.0156 and Δ*m*/*z* 14.0146 were detected for ergotamine (223.1239; 268.1440) compared to norergotamine (209.1077; 254.1294). The fragments correspond to the fragmentation of the amide bond between the lysergic acid and the tricyclic peptide ring. For these fragment ions, the observed difference in the *m*/*z* values between ergotamine and norergotamine correspond to the demethylation of the *N⁶*-atom (-CH₂; theoretical *m*/*z* 14.0156). The HR-masses of the precursor and product ions agreed with the theoretical values, giving us confidence that the electrochemical *N⁶*-demethylation of ergotamine had been achieved on an analytical scale.

However, significant challenges were encountered when attempting to scale up the electrochemical demethylation from a µg- to mg- scale. Approximately 85 % of the products formed by electrochemical demethylation of ergotamine are oxidation products. We observed primarily hydroxylation and the formation of double bonds at various positions. We therefore looked for an alternative method to N⁶ demethylation.

### Chemical demethylation

For the conversion of the tertiary amine (I) of ergotamine into its N-oxide (II) hydrogen peroxide (H₂O₂), meta-chloroperoxybenzoic acid (mCPBA) and potassium peroxymonosulfate (KHSO₅) were tested. However, H₂O₂ and KHSO₅ were found to be unsuitable due to incomplete conversion of ergotamine or significant by-product formation. The use of mCPBA resulted in the main oxidation product (II) with only minor by-product formation (See **Fig. 3****).**

The N-oxide can either be isolated or used directly in the second step. Therefore, an acid (such as sulfuric acid or hydrochloric acid) and an Fe^{II}-salt or Fe⁰ (iron powder) is added to the reaction mixture. The Fe⁰ is oxidized and forms the active Fe^{II} species *in situ.* A redox pair of Fe^{II}/ Fe^{III}, involving two consecutive one-electron transfers, is believed to be responsible for the sequential reduction of the N-oxide. We experimented with iron(II) sulfate, iron(II) chloride, ferrocene and iron powder in various solvents and concentrations for the reduction of ergotamine-N-oxide. Over a reaction time of 24-hours, the tested iron salts resulted in only minor product formation, while ferrocene primarily promoted the formation of ergotamine/-inine (I) as major by-product and only showed minor formation of norergotamine (III). When using iron powder in aprotic solvents (tetrahydrofuran and dichloromethane), we mainly obtained the parent tertiary amine (I). However, changing to a protic solvent like methanol resulted in substantial formation of the N-demethylated product. The highest conversion for the demethylation was achieved using iron-powder in a 10-fold excess. HR-MS/MS analysis of the formed demethylated product show a mass shift of 4 Da for specific fragment ions containing the N⁶-atom, which matches the HR-MS/MS spectra of the electrochemically synthesized product.

During the formation of the desired product, significant epimerization was observed for the N-demethylated product (III) and the main by-product / educt (I). This epimerization is known and believed to be caused by a combination of low pH, heat, and the use of a protic solvent during the reaction. Preparative HPLC was used to isolate both epimers, norergotamine and norergotaminine, in sufficient quantities for further reactions.

### Structural elucidation of Ergotamine- ¹³ CD₃ and Ergotaminine- ¹³CD₃

Methyliodide-¹³CD₃ was used to methylate the crude product of pure norergotamine and norergotaminine (See **Fig. 4****).** The resulting isotopically labeled ergotamine and ergotaminine were purified using preparative HPLC to remove any unreacted norergotamine/-inine. This process resulted in epimerically pure solutions of the isotopically labeled ergotamine and ergotaminine. To verify this, a sample of unlabeled ergotamine/-inine in methanol was spiked with isotopically labeled ergotamine/-inine and analyzed using HPLC-HR-MS/MS (See **Figs. 5** and **6****).**

As illustrated by **Figs. 5** and **6** a distinct mass shift of Δm=4 Da is observed in the isotopically labeled molecules, especially within the fragment ions containing the labeled *N⁶*-atom.

Surprisingly, the reaction of ergot alkaloids such as ergotamine/ergotaminine with mCPBA yielded mostly the desired oxidation products. The reduction of the oxidation product yielded the *N⁶*-demethylated ergotamine and ergotaminine. Subsequent reaction of norergotamine and norergotaminine with ¹³CD₃-I produced both isotopically labeled epimers of ergotamine. The data presented in **Figs. 5** and **6** confirm the structure of isotopically labeled ergotamine and ergotaminine.

### Electrochemical experiments

Ergotamine-D-tartrate (3,28 mg) was dissolved in 50 mL ACN or MeOH to give a stock solution of 100 µM ergotamine. NH₄OOCH₃ (315.3 mg), (NH₄)₂CO₃ (480.5 mg) and formic acid (188.7 µL) were placed in a 50 mL volumetric flask and filled up with ACN or MeOH to obtain a 100 µM stock solution. For the different experiments, 1 mL of the ergotamine-D-tartrate and the appropriate electrolyte/additive stock solutions were filled up to 10 mL with water and/or ACN/MeOH **(Table 1).** The reaction solution was pumped through the electrochemical cell with a velocity of 20 µL per minute and the potential of the potentiate was ramped between 0 and 1200 mV with at a scan rate of 5 mV/s. Direct measurements of the reaction products were carried out with the Agilent 6130 MS. For high resolution mass spectra, samples were collected and measured via direct infusion at the Sciex TripleTOF 6600.

### Synthesis of Norergotamine and Norergotaminine

Ergotamine-D-tartrate (10 mg, 15.2 µmol, 1.0 eq.) was suspended in 2 mL methanol and cooled down in an ice bath. After 15 min mCPBA (3.75 mg ,16.7 µmol, 1.1 eq) was added to the ice cooled suspension and the reaction was stirred for 1 hour at room temperature or until no ergotamine was detected by HPLC-MS. During the reaction, the solid particles dissolved, creating a homogeneous solution. The magnetic stir bar was re-moved and 1 molar hydrochloric acid in methanol (30.4 µL, 2.0 eq.), 5 g/L FeCl₃·6 H₂O (41.4 µL, 0.05 eq.) and iron powder (8.4 mg, 150.2 µmol, 9.9 eq) were added to the solution and shaken for 24 hours at 35 C. The color of the reaction changed from light yellow at the beginning to deep red at the end. After 24 hours, 1 mL of solvent was removed under a constant flow of nitrogen. The solution was basified with a solution of 0.5 molar trisodium phosphate in water (76 µL, 2.5 eq). The precipitate was removed by centrifugation and the supernatant was purified by preparative HPLC **(Table 2).**

**Table 2**

| Preparative HPLC conditions: Knauer Eurospher II 100-5 C18 P (250 x 4 mm; 5 µm) column, flow rate: 1 ml/min, column oven temperature: 35°C, injection volume: 100 µL, runtime: 31 min, eluents: H₂O + 20 mM NH₃, acetonitrile; DAD wavelength: 254 nm. | | |
|---|---|---|
| **Time [min]** | **H₂O + 20 mM NH₃ [%]** | **Acetonitrile [%]** |
| 0 | 66 | 34 |
| 20 | 66 | 34 |
| 20.1 | 0 | 100 |
| 25 | 0 | 100 |
| 25.1 | 66 | 34 |
| 31 | 66 | 34 |

The norergotamine/norergotamine fractions were combined and dried overnight in a rotary vacuum concentrator at 35°C and 11 mbar pressure. 1.5 mg of a mixture of norergotamine and norergotaminine were obtained.
*m*/*z* (measured) (M+H)⁺ = 568.2556 (theoretical (M+H)⁺: 568.2555 , δ = 0.2 ppm)

### Synthesis of Ergotamine-¹³CD₃ and Ergotaminine-¹³CD₃

A mixture of norergotamine and norergotaminine (1.5 mg, 2.6 µmol, 1.0 eq.) was dissolved in 300 µL acetone. *N,N*-Diisopropylethylamine (3.9 µmol, 1.5 eq.) and ¹³CD₃-I (3.9 µmol, 1.5 eq) were added to the solution and shaken at room temperature for 24 hours. The solvent was removed in a rotary vacuum concentrator and the residue was redissolved in 200 µL acetonitrile/ methanol/ water+20 mM NH₃ (40 v %/ 50 v %/ 10 v %). The crude mixture was purified via preparative HPLC **(Table 3)** to yield in total 1.33 mg (2.27 µmol) ergotamine-¹³CD₃ and ergotaminine-¹³CD₃.

**Table 3**

| Preparative HPLC conditions: Knauer Eurospher II 100-5 C18 P (250 x 4 mm; 5 µm) column, flow rate: 1 ml/min, column oven temperature: 35°C, injection volume: 100 µL, runtime: 33 min, eluents: H₂O + 20 mM NH₃, acetonitrile; DAD wavelength: 254 nm. | | |
|---|---|---|
| **Time [min]** | **H₂O** + **20 mM NH₃ [%]** | **Acetonitrile [%]** |
| 0 | 62 | 38 |
| 14 | 62 | 38 |
| 16 | 35 | 65 |
| 22 | 35 | 65 |
| 22. 1 | 0 | 100 |
| 27 | 0 | 100 |
| 27.1 | 62 | 38 |
| 33 | 62 | 38 |

*m*/*z* (measured) (M+H)⁺ = 586.2951 (theoretical (M+H)⁺ : 586.2933 , δ = 3.1 ppm).

In view of the ergotamine-¹³CD₃ and ergotamine-¹³CD₃ obtained, we conclude that isotopomers of all other priority ergot alkaloids mentioned above can be obtained using the same synthesis procedure. As illustrated by **Fig. 3** the suggested general synthesis procedure targets a shared structural feature among all priority ergot alkaloids.

To demonstrate this by way of example, we describe below the practical application of the synthesis strategy to four other ergot alkaloids, α-ergocryptine/-inine, ergosine/-inine, ergocornine/-inine and ergocristine/inine to obtain the corresponding N⁶-labeled ¹³CD₃ analogs.

### Synthesis of α-norersocryptine and α-norergocryptinine

α-Ergocryptine (11.1 mg, 19.3 µmol, 1.0 eq.) was dissolved in 2 mL dichloromethane and cooled down in an ice bath. After 15 min mCPBA (4.75 mg, 21.2 µmol, 1.1 eq) was added to the ice cooled solution and the reaction was stirred for 1 hour at room temperature or until no α-ergocryptine was detected by HPLC-MS. The magnetic stir bar was removed and 1 molar hydrochloric acid in methanol (38.6 µL, 2.0 eq.), 5 g/L FeCl₃·6 H₂O (52.2 µL, 0.05 eq.) and iron powder (10.4 mg, 186.2 µmol, 9.6 eq) were added to the solution and shaken for 3 hours at 35 C or until no *N⁶*-oxide of α-ergocryptine was detected by HPLC-MS. The color of the reaction changed from light yellow at the beginning to deep red-brown at the end. After the reaction was finished, the solvent was removed by blowdown evaporation and the residue was redissolved in 1 mL acetonitrile/water (70 v% / 30 v%). The solution was basified with a solution of 0.5 molar trisodium phosphate in water (96.5 µL, 2.5 eq). The precipitate was removed by centrifugation and the supernatant was purified by preparative HPLC to yield 3.9 mg (6.9 µmol) of α-norergocryptine and α-norergocryptinine **(****Fig. 8****).**

*m*/*z* (measured) (M+H)⁺ = 562.3036 (theoretical (M+H)⁺ : 562.3024 , δ = 2.1 ppm)

### Synthesis of α-Ergocryptine-¹³CD₃ and α-Ergocryptinine-¹³CD₃

A mixture of α-norergocryptine and α-norergocryptinine (3.9 mg, 6.9 µmol, 1.0 eq.) was dissolved in 500 µL acetone. *N,N*-Diisopropylethylamine (10.4 µmol, 1.5 eq.) and ¹³CD₃-I (10.4 µmol, 1.5 eq) were added to the solution and shaken at room temperature for 24 hours. The solvent was removed in a rotary vacuum concentrator and the residue was redissolved in 300 µL acetonitrile / water+20 mM NH₃ (80 v % / 20 v %). The crude mixture was purified via preparative HPLC **(Table 3)** to yield in total 3.3 mg (5.7 µmol) α-ergocryptine-¹³CD₃ and α-ergocryptinine-¹³CD₃ **(****Figs. 7** and **9****).**

m/z (measured) (M+H)⁺ = 576.3178 (theoretical (M+H)⁺ : 576.3180 , δ = -0.3 ppm)

### Synthesis of norergocristine and norergocristinine

Ergocristine (10.3 mg, 16.9 µmol, 1.0 eq.) was dissolved in 2 mL dichloromethane and cooled down in an ice bath. After 15 min mCPBA (4.16 mg, 18.6 µmol, 1.1 eq) was added to the ice cooled solution and the reaction was stirred for 1 hour at room temperature or until no ergocristine was detected by HPLC-MS. The magnetic stir bar was removed and 1 molar hydrochloric acid in methanol (33.8 µL, 2.0 eq.), 5 g/L FeCl₃·6 H₂O (46.6 µL, 0.05 eq.) and iron powder (9.6 mg, 171.9 µmol, 10.2 eq) were added to the solution and shaken for 3 hours at 35 C or until no *N⁶*-oxide of ergocristine was detected by HPLC-MS. The color of the reaction changed from light yellow at the beginning to deep red-brown at the end. After the reaction was finished, the solvent was removed by blowdown evaporation and the residue was redissolved in 1 mL acetonitrile/water (70 v% / 30 v%). The solution was basified with a solution of 0.5 molar trisodium phosphate in water (84.5 µL, 2.5 eq). The precipitate was removed by centrifugation and the supernatant was purified by preparative HPLC to yield 1.8 mg (3.0 µmol) of norergocristine and norergocristinine **(****Fig. 11****).**

m/z (measured) (M+H)⁺ = 596.2860 (theoretical (M+H)⁺ : 596.2867 , δ = -1.2 ppm)

### Synthesis of Ergocristine-¹³CD₃ and Ergocristinine-¹³CD₃

A mixture of norergocristine and norergocristinine (1.8 mg, 3.0 µmol, 1.0 eq.) was dissolved in 300 µL acetone. *N,N*-Diisopropylethylamine (4.5 µmol, 1.5 eq.) and ¹³CD₃-I (4.5 µmol, 1.5 eq) were added to the solution and shaken at room temperature for 24 hours. The solvent was removed in a rotary vacuum concentrator and the residue was redissolved in 200 µL acetonitrile / water+20 mM NH₃ (80 v % / 20 v %). The crude mixture was purified via preparative HPLC **(Table 3)** to yield in total 1.4 mg (2.3 µmol) ergocristine-¹³CD₃ and ergocristinine-¹³CD₃ **(****Figs. 10** and **12****).**

m/z (measured) (M+H)⁺ = 610.3026 (theoretical (M+H)⁺ : 610.3024 , δ = 0.3 ppm)

### Synthesis of norergocornine and norergocorninine

Ergocornine (10.0 mg, 17.8 µmol, 1.0 eq.) was dissolved in 2 mL dichloromethane and cooled down in an ice bath. After 15 min mCPBA (4.38 mg, 19.6 µmol, 1.1 eq) was added to the ice cooled solution and the reaction was stirred for 1 hour at room temperature or until no ergocornine was detected by HPLC-MS. The magnetic stir bar was removed and 1 molar hydrochloric acid in methanol (35.6 µL, 2.0 eq.), 5 g/L FeCl₃·6 H₂O (48.1 µL, 0.05 eq.) and iron powder (10.1 mg, 179.1 µmol, 10.1 eq) were added to the solution and shaken for 3 hours at 35°C or until no N⁶-oxide of ergocornine was detected by HPLC-MS. The color of the reaction changed from light yellow at the beginning to deep red-brown at the end. After the reaction was finished, the solvent was removed by blowdown evaporation and the residue was redissolved in 1 mL acetonitrile/water (70 v% / 30 v%). The solution was basified with a solution of 0.5 molar trisodium phosphate in water (89 µL, 2.5 eq). The precipitate was removed by centrifugation and the supernatant was purified by preparative HPLC to yield 2.4 mg (4.4 µmol) of norergocornine and norergocominine **(****Fig. 14****).**

m/z (measured) (M+H)⁺ = 548.2870 (theoretical (M+H)⁺ : 548.2868 , δ = 0.4 ppm)

### Synthesis of Ergocornine-¹³CD₃ and Ergocorninine-¹³CD₃

A mixture of norergocornine and norergocorninine (2.4 mg, 4.4 µmol, 1.0 eq.) was dissolved in 400 µL acetone. *N,N*-Diisopropylethylamine (6.6 µmol, 1.5 eq.) and ¹³CD₃-I (6.6 µmol, 1.5 eq) were added to the solution and shaken at room temperature for 24 hours. The solvent was removed in a rotary vacuum concentrator and the residue was redissolved in 250 µL acetonitrile / water+20 mM NH₃ (80 v % / 20 v %). The crude mixture was purified via preparative HPLC **(Table 3)** to yield in total 2.0 mg (3.5 µmol) ergocornine-¹³CD₃ and ergocominine-¹³CD₃ **(****Figs. 13** and **15****).**

m/z (measured) (M+H)⁺ = 566.3241 (theoretical (M+H)⁺ : 566.3246 , δ = -0.9 ppm)

### Synthesis of norergosine and norergosinine

Ergosine mesylate (5.0 mg, 7.8 µmol, 1.0 eq.) was suspended in 1 mL dichloromethane and 1 molar ammonia solution (8.6 µL, 8.6 µmol, 1.1 eq.) was added to the solution. The solution was vigorously shaken, and the solid particles dissolved, creating a homogeneous solution. After 10 minutes, the solvent was removed using a rotary vacuum concentrator. The residue was then redissolved in 1 mL of dichloromethane and centrifuged for 3 minutes at 14500 rpm. The resulting supernatant, which contained ergosine as a free base, was used for the subsequent reaction. The solution was cooled down in an ice bath and after 15 min mCPBA (1.74 mg, 8.6 µmol, 1.1 eq) was added to the ice cooled solution and the reaction was stirred for 1 hour at room temperature or until no ergosine was detected by HPLC-MS. The magnetic stir bar was removed and 1 molar hydrochloric acid in methanol (15.6 µL, 2.0 eq.), 5 g/L FeCl₃·6 H₂O (21.1 µL, 0.05 eq.) and iron powder (4.7 mg, 84.1 µmol, 10.8 eq) were added to the solution and shaken for 3 hours at 35°C or until no N⁶-oxide of ergosine was detected by HPLC-MS. The color of the reaction changed from light yellow at the beginning to deep red-brown at the end. After the reaction was finished, the solvent was removed by blowdown evaporation and the residue was redissolved in 500 µL acetonitrile/water (70 v% / 30 v%). The solution was basified with a solution of 0.5 molar trisodium phosphate in water (39 µL, 2.5 eq). The precipitate was removed by centrifugation and the supernatant was purified by preparative HPLC to yield of norergosine and norergosinine **(****Fig. 17****).**
m/z (measured) (M+H)⁺ = 534.2710 (theoretical (M+H)⁺ : 534.2711 , δ = -0.2 ppm)

### Synthesis of Ergosine-¹³CD₃ and Ergosinine-¹³CD₃

A mixture of norergosine and norergosinine was dissolved in 200 µL acetone. *N,N*-Diisopropylethylamine (2 µmol,) was added to the solution and subsequently small portions of ¹³CD₃-I in acetone (0.5 µmol/aliquote) was added to the solution and shaken at room temperature. After 1 hour the progress of the reaction was monitored with HPLC-MS and if substantial amounts of norergosine/-inine was detected, another aliquot was added. When only a minor amount of norergosine/-inine was detected with HPLC-MS, the solvent was removed in a rotary vacuum concentrator and the residue was redissolved in 250 µL acetonitrile / water + 20 mM NH₃ (80 v % / 20 v %). The crude mixture was purified via preparative HPLC **(Table 3)** to yield ergosine-¹³CD₃ and ergosinine-¹³CD₃ **(****Figs. 16** and **18****).**
m/z (measured) (M+H)⁺ = 552.3102 (theoretical (M+H)⁺ : 552.3089, δ = 2.3 ppm)

### Chemicals and Equipment

All chemicals were used without further purification. Ergotamine-D-tartrate, 3-chloroperoxybenzoic acid (mCPBA, ≤ 77 %), iron powder (for analysis, 10 µm), N,N-diisopropylethylamine (≥ 99 %) and ferrocene (for synthesis) were purchased from Sigma-Aldrich (Merck KGaA, Darmstadt, Germany). Iron(II) sulfate heptahydrate, iron(II) chloride tetrahydrate and tri-sodium phosphate-12-hydrate were bought from Riedel-de-Haën (Honeywell, Charlotte, North Carolina, USA). Ammonium formate (NH₄OOCH₃), Honeywell, Charlotte, North Carolina, USA), ammonium carbonate ((NH₄)₂CO₃), Thermo Fisher Scientific, Waltham, Massachusetts, USA) and formic acid (FA, Carlo Erba, Emmendingen, Germany) were bought as electrolytes. Methanol (MeOH), iso-propanol (iPrOH), dichloromethane (DCM), acetone (ACE), tetrahydrofurane (THF) and acetonitrile (ACN) were all HPLC-grade or higher and obtained from Th. Geyer (Renningen, Germany). Isotopic labeled methyl iodide (¹³CD₃-I) was bought from Eurisotop (Saint-Aubin, France).

The electrochemical experiments were performed in a three-electrode cell (µ-prepcell^{™}, Antec, Zoeterwoude, The Netherlands) connected to a ROXY^{™} potentiostat controlled using Dialogue software. The µ-prepcell consists of a working electrode (boron doped diamond (BDE), glassy carbon (GC) or platinum (PT)), a reference electrode (Pd/H2, HyREF^{™}) and an auxiliary electrode (conductive polymeric inlet block). The reaction solution was pumped through the flow-cell at flow rate of 20 µL/min with a syringe pump (KD Scientific Inc., Holliston, MA, USA). The electrochemical cell was connected to a 6130 quadrupole MS (Agilent, Waldbronn, Germany).

Measurements for reaction control were carried out on an Agilent 1290 Infinity HPLC system (Agilent, Waldbronn, Germany) coupled to a 6130 quadrupole MS (Agilent, Waldbronn, Germany). For the separation of reaction products, a Phenomenex Gemini-NX C18 (150 × 2.0 mm; 3 µm) column was used.

Preparative HPLC was performed on an Agilent (Waldbronn, Germany) HPLC-system consisting of 1260 Infinity quaternary pump, 1200 Autosampler and column oven coupled to an Agilent 1200 Diode Array Detector with the wavelength set to 254 nm. The Foxy R1 Fraction Collector (Teledyne Isco, Lincoln, Nebraska, United States of America) was used to automate sample fractionation. A Knauer Eurospher II 100-5 C18 P (250 × 4 mm; 5 µm) column was used.

Volatile solvents were removed by nitrogen blow down in a Reacti-Therm^{™} Heating and Stirring Module (Thermo Fischer Scientific, Waltham, Massachusetts, USA). Purified products were dried in a Rotary Vacuum Concentrator RVC 2-25 CDplus (Christ, Osterode am Harz, Germany). For thermoshaking, an HLC MHR-13 thermoshaker (Hettich, Tübingen, Germany) was used.

### HPLC-HR-MS/MS

High-resolution mass spectra were measured on TripleTOF 6600 mass spectrometer (Sciex, Darmstadt, Germany) coupled to a 1290 Infinity II system (Agilent, Waldbronn, Germany). As indicated in **Table 4** for the measurements, a Phenomenex Gemini C18 (50 × 2 mm; 3 µm) column was used.

**Table 4**

| HPLC conditions: Phenomenex Gemini C18 (50 x 2 mm; 3 µm) column, flow rate: 0.5 ml/min, column oven temperature: 35°C, injection volume: 5 µL, runtime: 15 min, eluents: H₂O + 20 mM NH₃, acetonitrile. | | |
|---|---|---|
| **Time [min]** | **H₂O + 20 mM NH₃ [%]** | **Acetonitrile [%]** |
| 0 | 70 | 30 |
| 1 | 70 | 30 |
| 10 | 30 | 70 |
| 10.1 | 10 | 90 |
| 12 | 10 | 90 |
| 12.1 | 70 | 30 |
| 15 | 70 | 30 |

**Table 5** presents the TripleTOF conditions. MS/MS experiments were conducted using information-dependent acquisition (IDA). The MS/MS spectra were recalibrated based on the theoretical *m*/*z* values of the precursor ion. Formulae for the measured product ion masses were calculated using a threshold of ±5 ppm deviation.

**Table 5**

| Parameters for the TripleTOF 6600 ESI-HR-MS/MS measurements. | | |
|---|---|---|
| **Parameter** - **ESI Source** | **Parameters** - **Mass spectrometer** | |
| Temperature: 300 °C | MS1 | |
| | Collision energy | 5 V |
| Ion Source Gas 1: 60 L/min | Declustering Potential | 80 V |
| Ion Source Gas 2: 70 L/min | Mass range | 400 - 800 |
| Curtain Gas: 25 L/min | MS 2 | |
| Ionspray Voltage: 5500 V | Collision Energy | 40 |
| | Collision Energy Spread | 15 V |
| | Declustering Potential | 80 V |
| | Mass range | 100 - 800 |

Thus, for the first time, we were able to prepare isotopically labeled ergot alkaloids and their epimers starting from unlabeled ergot alkaloids in a simple two-step procedure. We are therefore confident that the proven synthesis of isotopically labeled ergotamine and ergotaminine, ergocristine and ergocristinine, ergocornine and ergocorninine, ergosine and ergosinine, and α-ergocryptine and α-ergocryptinine demonstrates the applicability of the two-step synthesis procedure used to obtain all required isotopically labeled analogs of all practically relevant ergot alkaloids to be synthesized on a preparative scale, since the discussed ergot alkaloids share a common structural feature: the methyl group at N⁶ which is the targeted of the proposed synthesis.

The present invention has been explained with reference to various illustrative embodiments and examples. These embodiments and examples are not intended to restrict the scope of the invention, defined by the claims and their equivalents. As apparent to one skilled in the art, the embodiments described herein can be implemented in various ways without departing from the scope of what is invented. The embodiments can freely be combined.

## Claims

1. A process of synthesizing an N⁶-isotope-labeled ergot alkaloid, comprising:
- demethylating an ergot alkaloid to obtain a corresponding N⁶-demethylated norergot alkaloid; and
- remethylating the corresponding N⁶-demethylated norergot alkaloid to obtain the N⁶-isotopically labeled ergot alkaloid;
wherein remethylating is carried out with an isotopically labeled methylation reagent.

2. The process according to claim 1,
wherein the demethylating is carried out in the presence of an iron-containing catalyst,
wherein the iron-containing catalyst comprises any of an Fe(0), Fe(2+), Fe(3+), and a porphyrin iron (2+/3+) complex (i.e. an iron porphyrinate).

3. The process according to claim 2,
wherein the iron-containing catalyst is an iron porphyrinate selected from:
iron(2+/3+) tetrabenzotetraazaporphyrin, iron(2+/3+) tetraazaporphyrin,
iron(2+/3+) trabenzoporphyrin and iron(2+/3+) porphyrin according to the scheme below:

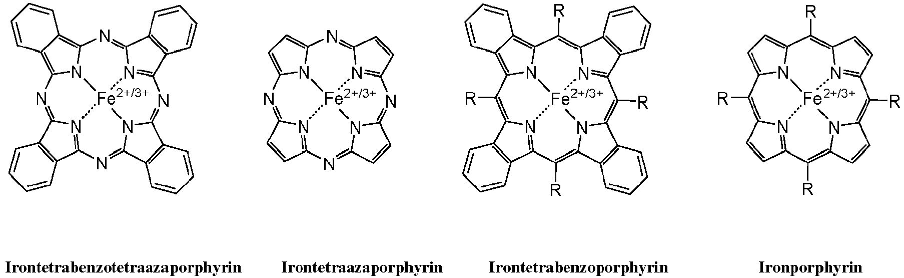

4. The process according to claim 3, wherein R is selected from: 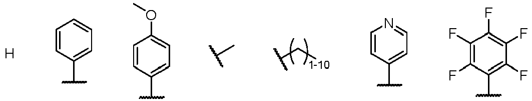 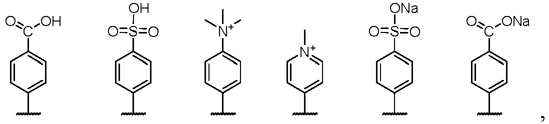 preferably from: 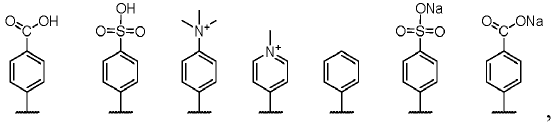 more preferably from:

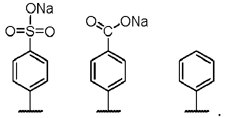

5. The process according to claim 1 or 2,
wherein the isotope-labeled methylating reagent is selected from the group consisting of ¹²C and ¹³C analogs of the following substances:
acetic acid;
iodomethane or methyl iodide and its derivatives, such as e.g. bromomethane;
dimethyl carbonate;
dimethyl sulfate;
dimethyl sulfite;
formaldehyde;
formic acid;
dimethyl sulfate;
dimethyl sulfoxide;
dimethyl carbonate;
methanol;
methyl-fluorosulfonate;
methyl- trifluoromethane sulfonate; and
p-toluene sulfonic acid methylester
trimethyloxonium tetrafluoroborate;
wherein hydrogen (H) of these substances is substituted by deuterium (D).

6. The process according to any of claims 1-5,
wherein the ergot alkaloid is selected from any of the diastereoisomer pairs of:
ergocornine/ergocorninine;
ergometrine/ergometrinine,
ergocristine/ergocristinine,
ergotamine/ergotaminine,
ergosine/ergosinine,
α-ergocryptine/ α-ergocryptinine, and
β-ergocryptine/β-ergocryptinine.

7. The process according to any of preceding claims,
wherein the ergot alkaloid comprises a *5R, 8R* configuration and/or a *5R, 8S* configuration of: ergotamine, ergocristine, ergocornine, ergosine and/or α-ergocryptine.

8. The process according to any of previous claims,
wherein the provided ergot alkaloid is a biosynthetically generated ergot alkaloid, in particular a native ergot alkaloid extracted from a sclerotium of *Claviceps spec.* and/or isolated from a cell culture of a microorganism.

9. The process according to any of previous claims,
wherein a mass shift Δm of the synthesized ergot alkaloid, which can be used in a mass spectrometry as internal standard in comparison to the natural ergot alkaloid is in a range of 3 ≤ Δm ≤ 6.

10. A method for detecting an ergot alkaloid in a sample, comprising determining a N⁶-isotope-labeled ergot alkaloid, wherein determining encompasses a mass spectrometry of the sample, of an extract of the sample, or of an aliquot of the sample, selected from the group consisting of:
- a mass spectrometric analysis of the sample, such as HPLC-MS or tandem mass spectrometry analysis, using a N⁶-isotope-labeled ergot alkaloid as an internal standard, and
- a stable isotope dilution analysis (SIDA) such as SIDA-HPLC-MS or MS/MS; wherein the N⁶-isotope-labeled ergot alkaloid is synthesized according to any of claims 1 - 9.

11. A N⁶-isotope-labeled ergot alkaloid for use as internal standard in a mass spectrometry analysis of a sample which is potentially contaminated by an ergot alkaloid, wherein the ergot alkaloid is selected from the group consisting of:
a *5R*, *8R* configuration of ergocornine, a *5R, 8S* configuration of ergocornine,
a *5R, 8R* configuration of ergometrine, a *5R, 8S* configuration of ergometrine,
a *5R, 8R* configuration of ergocristine , a *5R, 8S* configuration of ergocristine ,
a *5R, 8R* configuration of ergotamine , a *5R, 8S* configuration of ergotamine,
a *5R, 8R* configuration of ergosine, a *5R, 8S* configuration of ergosine,
a *5R, 8R* configuration of α-ergocryptine, a *5R, 8S* configuration of α-ergocryptine,
a *5R, 8R* configuration of β-ergocryptine, and a *5R, 8S* configuration of β-ergocryptine.

12. The N⁶-isotope-labeled ergot alkaloid according to claim 11,
wherein the isotope-label comprises any of ¹¹C, ¹²C, ¹³C, ¹⁴C, ¹H (hydrogen),
²H (deuterium), and ³H (tritium).

13. A kit for detection of a mycotoxin by using a mass spectrometry, comprising at least one N⁶-isotope-labeled ergot alkaloid according to any of claims 11 or 12,
wherein the ergocornine, the ergometrine, the ergocristine, the ergotamine, the ergosine, the α-ergocryptine, and the β-ergocryptine, whether in their *5R, 8R* configuration and/or in their *5R, 8S* configuration, are synthesized according to any of claims 1-9.

14. The kit according to claim 13,
wherein the kit comprises at least one vial containing at least one of the ergot alkaloids in a dry or otherwise stabilized form, wherein a quantity of the dry or otherwise stabilized ergot alkaloid in the vial is adapted to allow generating a defined concentration of said ergot alkaloid by dissolving and/or diluting it with a defined liquid volume.

15. Using an N⁶-isotope-labeled ergot alkaloid as an internal standard in a mass spectrometry measurement,
wherein the N⁶-isotope-labeled ergot alkaloid is produced by the method according to any of claims 1 - 9.

## Amended claims

### Amended claims in accordance with Rule 137(2) EPC.

1. A process of synthesizing an N⁶-isotope-labeled ergot alkaloid, comprising:
- providing a native ergot alkaloid extracted from a sclerotium of *Claviceps spec.* and/or isolated from a cell culture of a microorganism;
- demethylating an ergot alkaloid to obtain a corresponding N⁶-demethylated norergot alkaloid; and
- remethylating the corresponding N⁶-demethylated norergot alkaloid to obtain the N⁶-isotopically labeled ergot alkaloid;
wherein remethylating is carried out with an isotopically labeled methylation reagent.

2. The process according to claim 1,
wherein the demethylating is carried out in the presence of an iron-containing catalyst,
wherein the iron-containing catalyst comprises any of an Fe(0), Fe(2+), Fe(3+), and a porphyrin iron (2+/3+) complex (i.e. an iron porphyrinate).

3. The process according to claim 2,
wherein the iron-containing catalyst is an iron porphyrinate selected from:
iron(2+/3+) tetrabenzotetraazaporphyrin, iron(2+/3+) tetraazaporphyrin, iron(2+/3+) trabenzoporphyrin and iron(2+/3+) porphyrin according to the scheme below:

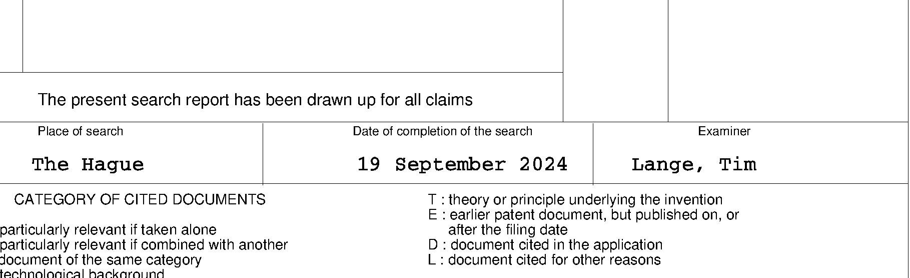

4. The process according to claim 3, wherein R is selected from: 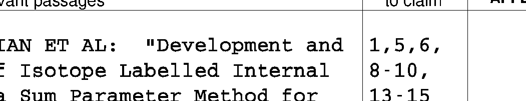 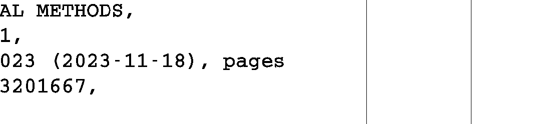 preferably from: 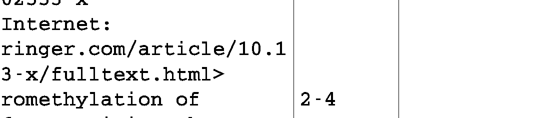 more preferably from:

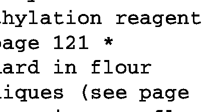

5. The process according to claim 1 or 2,
wherein the isotope-labeled methylating reagent is selected from the group consisting of ¹²C and ¹³C analogs of the following substances:
acetic acid;
iodomethane or methyl iodide and its derivatives, such as e.g. bromomethane;
dimethyl carbonate;
dimethyl sulfate;
dimethyl sulfite;
formaldehyde;
formic acid;
dimethyl sulfate;
dimethyl sulfoxide;
dimethyl carbonate;
methanol;
methyl-fluorosulfonate;
methyl- trifluoromethane sulfonate; and
*p*-toluene sulfonic acid methylester
trimethyloxonium tetrafluoroborate;
wherein hydrogen (H) of these substances is substituted by deuterium (D).

6. The process according to any of claims 1 - 5,
wherein the ergot alkaloid is selected from any of the diastereoisomer pairs of:
ergocornine/ergocorninine;
ergometrine/ergometrinine,
ergocristine/ergocristinine,
ergotamine/ergotaminine,
ergosine/ergosinine,
α-ergocryptine/ α-ergocryptinine, and
β-ergocryptine/β-ergocryptinine.

7. The process according to any of preceding claims,
wherein the ergot alkaloid comprises a *5R, 8R* configuration and/or a *5R, 8S* configuration of: ergotamine, ergocristine, ergocornine, ergosine and/or α-ergocryptine.

8. The process according to any of previous claims,
wherein a mass shift Δm of the synthesized ergot alkaloid, detectable in a mass spectrometry in comparison to the natural ergot alkaloid is in a range of 3 ≤ Δm ≤ 6.

9. A method for detecting an ergot alkaloid in a sample, comprising determining a N⁶-isotope-labeled ergot alkaloid, wherein determining encompasses a mass spectrometry of the sample, of an extract of the sample, or of an aliquot of the sample, selected from the group consisting of:
- a mass spectrometric analysis of the sample, such as HPLC-MS or tandem mass spectrometry analysis, using a N⁶-isotope-labeled ergot alkaloid as an internal standard, and
- a stable isotope dilution analysis (SIDA) such as SIDA-HPLC-MS or MS/MS; wherein the N⁶-isotope-labeled ergot alkaloid is synthesized according to any of claims 1 - 8.

10. A N⁶-isotope-labeled ergot alkaloid for use as internal standard in a mass spectrometry analysis of a sample which is potentially contaminated by an ergot alkaloid, wherein the ergot alkaloid is selected from the group consisting of:
*a 5R, 8R* configuration of ergocornine, a *5R, 8S* configuration of ergocornine,
*a 5R, 8R* configuration of ergometrine, a *5R, 8S* configuration of ergometrine,
*a 5R, 8R* configuration of ergocristine , a *5R, 8S* configuration of ergocristine ,
*a 5R, 8R* configuration of ergotamine , a *5R, 8S* configuration of ergotamine,
*a 5R, 8R* configuration of ergosine, a *5R, 8S* configuration of ergosine,
*a 5R, 8R* configuration of α-ergocryptine, a *5R, 8S* configuration of α-ergocryptine,
*a 5R, 8R* configuration of β-ergocryptine, and a *5R, 8S* configuration of β-ergocryptine.

11. The N⁶-isotope-labeled ergot alkaloid according to claim 10, wherein the ergot alkaloid is manufactured according to any of claims 1 to 9.

12. The N⁶-isotope-labeled ergot alkaloid according to claim 10,
wherein the isotope-label comprises any of ¹¹C, ¹²C, ¹³C, ¹⁴C, ¹H (hydrogen), ²H (deuterium), and ³H (tritium).

13. A kit for detection of a mycotoxin by using a mass spectrometry, comprising at least one N⁶-isotope-labeled ergot alkaloid according to any of claims 10 to 12,
wherein the ergocornine, the ergometrine, the ergocristine, the ergotamine, the ergosine, the α-ergocryptine, and the β-ergocryptine, whether in their *5R, 8R* configuration and/or in their *5R, 8S* configuration, are synthesized according to any of claims 1 - 8.

14. The kit according to claim 13,
wherein the kit comprises at least one vial containing at least one of the ergot alkaloids in a dry or otherwise stabilized form, wherein a quantity of the dry or otherwise stabilized ergot alkaloid in the vial is adapted to allow generating a defined concentration of said ergot alkaloid by dissolving and/or diluting it with a defined liquid volume.

15. Use of an N⁶-isotope-labeled ergot alkaloid as an internal standard in a mass spectrometry measurement,
wherein the N⁶-isotope-labeled ergot alkaloid is produced by the method according to any of claims 1 - 8.
